# EUROPEAN PATENT SPECIFICATION

(11) **EP 1 729 817 B1**
(45) Date of publication and mention of the grant of the patent: **05.03.2014**
(21) Application number: 05856302.4
(22) Date of filing: 30.03.2005
(51) Int. Cl.: A61K 48/00, A61K 38/00, A61P 35/00

(54) **GENE DELIVERY SYSTEM CONTAINING RELAXIN GENE AND PHARMACEUTICAL COMPOSITION USING RELAXIN**
RELAXIN-GEN ENTHALTENDES GENVERABREICHUNGSSYSTEM UND PHARMAZEUTISCHE ZUSAMMENSETZUNG, DIE RELAXIN VERWENDET
SYSTEME DE DELIVRANCE DE GENE CONTENANT LE GENE DE LA RELAXINE ET COMPOSITION PHARMACEUTIQUE UTILISANT LA RELAXINE

(30) Priority: 30.03.2004 KR 20040021601
(43) Date of publication of application: 13.12.2006
(73) Proprietor: Industry-Academic Cooperation Foundation, Seodaemun-gu Seoul 120-749 (KR)
(72) Inventor: YUN, Chae-Ok, Seoul 137-807 (KR); KIM, Joo-Hang, Seoul 158-055 (KR)
(74) Representative: Viering, Jentschura & Partner Patent- und Rechtsanwälte
(86) International application number: PCT/KR2005/000921
(87) International publication number: WO 2006/075819

(56) References cited:
- WO-A-99/40929
- US-A1- 2002 019 349
- SILVERTOWN JOSH D ET AL: "Adenovirus-mediated expression of human prorelaxin promotes the invasive potential of canine mammary cancer cells." ENDOCRINOLOGY, vol. 144, no. 8, August 2003 (2003-08), pages 3683-3691, XP002480991 ISSN: 0013-7227
- KIM J ET AL: "EVALUATION OF E1B GENE-ATTENUATED REPLICATING ADENOVIRUSES FOR CANCER GENE THERAPY" CANCER GENE THERAPY, NORWALK, CT, US, vol. 9, 1 January 2002 (2002-01-01), pages 725-736, XP008057984 ISSN: 0929-1903
- HALEY J ET AL: "PORCINE RELAXIN GENE STRUCTURE AND EXPRESSION" JOURNAL OF BIOLOGICAL CHEMISTRY, AL, vol. 262, no. 26, 1 September 1987 (1987-09-01), pages 11940-11946, XP002943484 ISSN: 0021-9258
- KIM JOO-HANG ET AL: "Relaxin expression from tumor-targeting adenoviruses and its intratumoral spread, apoptosis induction, and efficacy" JOURNAL OF THE NATIONAL CANCER INSTITUTE (CARY), vol. 98, no. 20, October 2006 (2006-10), pages 1482-1493, XP002480992 ISSN: 0027-8874
- UNEMORI E N ET AL: "RELAXIN INDUCES AN EXTRACELLULAR MATRIX-DEGRADING PHENOTYPE IN HUMAN LUNG FIBROBLASTS IN VITRO AND INHIBITS LUNG FIBROSIS IN A MURINE MODEL IN VIVO" JOURNAL OF CLINICAL INVESTIGATION, NEW YORK, NY, US, vol. 98, no. 12, 1 January 1996 (1996-01-01), pages 2739-2745, XP009066651 ISSN: 0021-9738
- ZHANG ET AL.: 'Relaxin activates the MAP kinase pathway in human endometrial stromal cells' J. CELL. BIOCHEM. vol. 85, no. 3, 2002, pages 536 - 544, XP008096331
- HOMBACH-KLONISCH ET AL.: 'INSL-3 is expressed in human hyperplastic and neoplastic thyrocytes' INT. J. ONCOL. vol. 22, no. 5, May 2003, pages 993 - 1001, XP009079252
- HOMBACH-KLONISCH ET AL.: 'Relaxin-like factor (RLF) is differentially expressed in the normal and neoplastic human mammary gland' CANCER vol. 89, no. 11, December 2000, pages 2161 - 2168, XP008095822
- STEMMERMANN ET AL.: 'Immunocytochemical identification of a relaxin-like protein in gastrointestinal epithelium and carcinoma: a preliminary report' J. ENDOCRINOL. vol. 140, no. 2, February 1994, pages 321 - 325, XP008096325

## Description

### FIELD OF THE INVENTION

The present disclosure relates to a novel gene delivery system and recombinant adenovirus, in particular, to a novel gene delivery system and recombinant adenovirus comprising the relaxin-encoding sequence, a pharmaceutical anti-tumor composition comprising the recombinant adenovirus, a pharmaceutical composition characterized by improved tissue penetration potency and a pharmaceutical composition for treating a disease or disorder associated with accumulation of excess extracellular matrix.

### DESCRIPTION OF THE RELATED ART

Gene therapy is directed to the treatment of a pathological condition by introducing an exogenous gene into cells or tissues. For hereditary diseases such as sickle cell anemia, α₁ antitrypsin deficiency, phenylketonuria, hemophilia and cystic fibrosis, the aim of gene therapy is to replace a missing or defective gene in order to allow a cell or tissue to function normally. In addition, gene therapy is used to remove aberrant cells. Gene therapy permits to treat various diseases such as cancer, inflammation and autoimmune diseases by delivering genes capable of causing the death of target aberrant cells.

In spite of the promise of gene therapy, inefficient delivery of genes to cells or tissues remains a major obstacle in the development of a successful gene therapy. For example, a number of researches on gene delivery using retrovirus, adenovirus or adeno-associated viruses (AAV) have showed insufficient gene delivery efficiency when applied to individual or tissues (e.g., tumor tissues), discouraging the application of gene therapy.

Therefore, a novel gene delivery strategy exhibiting improved gene delivery efficiency remains essential for accomplishing a successful gene therapy.

Early adenovirus-based gene therapy usually employs replication-incompetent adenoviruses carrying a therapeutic gene with deleted E1 gene essential for adenovirus replication. However, these recombinant adenoviruses induce anti-tumor activity only in infected cells and a very small number of surrounding cells, exhibiting serious problems 'in clinical applications.

To overcome such problems, the oncolytic adenovirus, ONYX-015(d11520) selectively replicating in tumor cells has been developed. The EIB 55 kDa gene-deleted adenovirus selectively replicates in tumor cells lacking functional p53. When the recombinant adenovirus infects normal cells, its proliferation is inhibited to result in the failure of oncolysis because p53 inactivation is not induced, whereas it actively proliferates in tumor cells with inactivated p53 and eventually leads to selective death of tumor cells (Chang, F., et al., J Clin Oncol 13:1009-22(1995)).

According to recent reports on Phase-II/III clinical trials for brain cancer, a tumor-specific oncolytic adenovirus exhibits considerable therapeutic efficacy (Kirn, D., et al., Nat Med 4:1341-2(1998) ; Nemunaitis, J. et al., Cancer Res 60:6359-66(2000); and Ganly, I. et al., Clin Cancer Res 6:798-806(2000)). Although the administration of the recombinant adenovirus induces the partial suppression of tumor growth, the complete eradiation of tumor does not been found and regrowth of tumor rapidly occurs after the lapse of a period of time. Theses results are probably because the recombinant adenovirus topically injected into tumor are partially spread within a limited surrounding portion to elicit a restricted anti-tumor activity such that tumor cells not infected with viruses rapidly grow. According to a recent research report, the recombinant adenoviruses administered into human tumor in nude mice persistently replicate as late as 100 days after initial viral injection and do not ensure the complete eradication of tumor, while viable viruses may be obtained from tumor tissue (Sauthoff, H. et al., Human Gene Therapy 14:425-433(2003)).

Consequently, it could be appreciated that the ideal tumor-specific oncolytic adenovirus has the ability to induce greater oncolytic activity and spread throughout tumor tissue as well for infecting surrounding tumor cells.

### DETAILED DESCRIPTION OF THIS INVENTION

The present inventors have made intensive researches to improve the transduction efficiency of gene delivery systems, particularly, to enhance the transduction (or spreading) efficiency of gene delivery systems in tissues, as a result, discovering that relaxin could dramatically improve the transduction efficiency of gene delivery systems.

Accordingly, it is an object of this disclosure to provide a gene delivery system with improved transduction efficiency.

It is another object of this invention to provide a method for delivering a gene into cells with improved transduction efficiency.

It is still another object of this disclosure to provide a recombinant adenovirus having improved abilities in tumor tissue penetration and tumor-specific apoptosis.

It is further object of this disclosure to provide a pharmaceutical anti-tumor composition for treating a cancer.

It is still further object of this invention to provide a method for treating a cancer by use of the pharmaceutical anti-tumor composition.

It is another object of this disclosure to provide a pharmaceutical composition for improving a penetration potency of a medicament into a tissue.

It is still another object of this invention to provide a pharmaceutical composition for treating a disease or condition associated with accumulation of excess extracellular matrix.

Other objects and advantages of the present invention will become apparent from the detailed description to follow taken in conjugation with the appended claims and drawings.

In one aspect of this disclosure there is provided a gene delivery system comprising a nucleotide sequence of interest to be delivered into a cell, the improvement which comprises a relaxin-encoding nucleotide sequence to enhance a transduction efficiency of the nucleotide sequence of interest into the cell.

The present inventors have made intensive researches to improve the transduction efficiency of gene delivery systems, particularly, to enhance the transduction (or spreading) efficiency of gene delivery systems in tissues. Such researches are based on our hypothesis that the reduction in the level of components of extracellular matrix by facilitating the degradation or inhibiting the synthesis of components of extracellular matrix may enhance the spreading of gene delivery systems within tissues. Surprisingly, the present inventors have discovered that relaxin could dramatically improve the transduction efficiency of gene delivery systems.

The term "relaxin" used herein encompasses relaxin illustrated and exemplified in Examples as well as its any homologue to enhance transduction efficiency that is intent of the present invention.

Relaxin, that plays a pivotal role as an enhancer in improving transduction efficiency in the present invention, is a 6 kDa peptide hormone, structurally related to insulin and insulin-like growth factors (IGFs). It is predominantly produced in corpus luteum and endometrium and its serum level greatly increases during pregnancy (Sherwood, O.D. et al., Dynamic changes of multiple forms of serum immunoactive relaxin during pregnancy in the rat. Endocrinology 114:806-13(1984)). While relaxin was initially classified as "pregnancy hormone" based on earlier studies to report that it was active only in sex organs during pregnancy, it was recently known as "master hormone" because its activity was found in other organs and tissues than sex organs (Hisaw, F. L., et al., Effects of relaxin on the endothelium of endometrial blood vessels in monkeys (Macaca mulatta). Endocrinology 81:375-85(1967)).

Relaxin is known to facilitate the growth and regeneration of placenta and uterus and loosen of uterine cervix to broaden birth canal during parturition. It promotes the expression of various MMPs in birth canal tissues such as MMP2, MMP3 and MMP9 to degrade collagen, so that connective tissues and basal membranes are degraded to lead to the disruption of extracellular matrix of birth canal. In addition to this, the promotion of MMP 1 and MMP 3 expressions by relaxin is also observed in lung, heart, skin, intestines, mammary gland, blood vessel and spermiduct where relaxin plays a role as an inhibitor to prevent overexpression of collagen (Qin, X., et al., Biol Reprod 56:800-11(1997); Qin, X., et al., Biol Reprod 56:812-20(1997); and Palejwala, S. et al., Endocrinology 142:3405-13(2001)).

According to the gene delivery system of the present invention, the relaxin protein expressed induces the degradation of collagen, a major component of extracellular matrix surrounding cells, to disrupt connective tissue and basal membrane, thereby resulting in the degradation of extracellular matrix. This successive action is one of mechanisms underlying the improvement in transduction efficiency by relaxin, which is clearly verified in Examples described below.

Therefore, referring to the above-described action of relaxin, the advantages of the present gene delivery system is highlighted for cells within tissues composed of cells interconnected each other by extracelluar matrix. In particular, where applied to tumor tissues enclosed tightly by connective tissue, the present gene delivery system exhibits improved transduction efficiency compared to any conventional delivery system.

The term "gene delivery" used herein refers to the transfer of gene into cells and has the same meaning as gene transduction. In tissue level, the gene delivery becomes the same meaning as spread of gene. Therefore, the gene delivery system of this invention is also expressed as either gene transduction system or gene spreading system.

To construct the present gene delivery system, it is preferred that the relaxin-encoding nucleotide sequence is contained in a suitable expression construct. According the expression construct, it is preferred that the relaxin-encoding nucleotide sequence is operatively linked to a promoter. The term "operatively linked" refers to functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence. According to the present invention, the promoter linked to the relaxin gene is operable in, preferably, animal, more preferably, mammalian cells, to control transcription of the relaxin gene, including the promoters derived from the genome of mammalian cells or from mammalian viruses, for example, CMV (cytomegalovirus) promoter, the adenovirus late promoter, the vaccinia virus 7.5K promoter, SV40 promoter, HSV tk promoter, RSV promoter, EF1 alpha promoter, metallothionein promoter, beta-actin promoter, human IL-2 gene promoter, human IFN gene promoter, human IL-4 gene promoter, human lymphotoxin gene promoter and human GM-CSF gene promoter. Most preferably, the promoter is CMV promoter.

Preferably, the expression construct used in this invention comprises a polyadenylation sequence (e.g., bovine growth hormone terminator and SV40-derived polyadenylation sequence).

According to a preferred embodiment, the expression construct for the relaxin-encoding nucleotide sequence has a structure of "promoter-relaxin-encoding nucleotide sequence-polyadenylation sequence.

In the present gene delivery system, the nucleotide sequence of interest to be delivered into cells may be contained in an expression construct having the same structure for that for the relaxin-encoding nucleotide sequence.

The nucleotide sequence of interest to be delivered into cells may be any sequence, for example, including cancer-therapeutic genes encoding proteins having anti-tumor activity and eventually degenerating tumor cells such as tumor suppressor genes, immunomodulatory genes [e.g, cytokine genes, chemokine genes and costimulatory factor genes (for T cell activity such as B7.1 and B7.2)], antigenic genes, suicide genes, cytotoxic genes, cytostatic genes, pro-apoptotic genes and anti-angiogenic genes, but not limited to.

The suicide genes encode proteins capable of conferring to tumor cells sensitivity to chemotherapeutic agents, or of inducing toxic conditions in tumor cells. The most well-known suicide gene is the herpes simplex virus-thymidine kinase (HSV-TK) gene (U.S. Pat. Nos. 5,631,236 and 5,601,818). Cells expressing HSV-TK are susceptible to selective cell death by gancyclovir. The tumor suppressor genes encode polypeptides to inhibit tumorigenesis. The tumor suppressor genes are inherent in mammalian cells and their deletion or inactivation is believed to be a prerequisite for tumorigenesis. Examples of the tumor suppressor genes include members of the tumor suppressor gene INK4 family, which are exemplified by APC, DPC4, NF-1, NF-2, MTS1, WT1, BRCA1, BRCA2, VHL, p53, Rb, MMAC-1, MMSC-2, retinoblastoma gene (Lee et al., Nature, 329:642(1987)), gene of adenomatous polyposis coli protein (Albertsen et al., U.S. Pat. No. 5783,666), nasopharyngeal carcinoma tumor suppressor gene that maps at chromosome 3p21.3 (Cheng et al., Proc. Natl. Acad. Sci., 95:3042-3097(1998)), deleted in colon carcinoma (OCC) gene, MTS1, CDK4, VHL, p100Rb, p16 and p21, and and therapeutically effective fragments thereof (e.g., p56Rb, p94Rb). It will be understood that other known anti-tumor genes can be used by those of ordinary skill in the art.

The term "antigenic genes" as used herein, refers to a nucleotide sequence coding for antigenic cell surface protein which is recognized by the immune system. Examples of the antigenic genes include carcinoembryonic antigen (CEA), prostate specifc antigen (PSA), α-feto protein (AFP) and p53 (WO 94/02167). In order to facilitate immune recognition, the antigenic gene may be fused to the MHC type I antigen.

The term "cytotoxic gene" as used herein, refers to a nucleotide sequence, the expression of which in a cell elicits a toxic effect. Examples of the cytotoxic genes include nucleotide sequences encoding Pseudomonas exotoxin, ricin toxin, diphtheria toxin and the like.

The term "cytostatic gene" as used herein, refers to a nucleotide sequence, the expression of which in a cell induces an arrest in the cell cycle. Examples of the cytostatic genes include, but are not limited to, p21, retinoblastoma gene, E2F-Rb fusion protein gene, genes encoding cyclin-dependent kinase inhibitors such as p16, p15, p18 and p19, growth arrest specific homeobox (GAX) gene (W0 97/16459 and W0 96/30385).

In addition, a variety of therapeutic genes useful in treating various diseases may be carried in the gene delivery system of this invention. Non-limiting examples of the therapeutic genes include genes encoding cytokines (e.g., interferon-α, interferon-β, interferon-δ and interferon-γ), interleukin (e.g., IL-1, IL-2, IL-4, IL-6, IL-7, IL-10, IL-12, IL-19 and IL-20), colony-stimulating factors (e.g., GM-CSF and G-CSF), chemokine genes [monocyte chemotactic protein 1 (MCP-1), monocyte chemotactic protein 2 (MCP-2), monocyte chemotactic protein 3 (MCP-3), monocyte chemotactic protein 4 (MCP-4), macrophage inflammatory protein 1α (MIP-1α), macrophage inflammatory protein 1β (MIP-1β), macrophage inflammatory protein 1 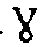 (MIP-1 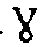 ), macrophage inflammatory protein 3α (MIP-3α), macrophage inflammatory protein 3β (MIP-3β), chemokine (ELC), macrophage inflammatory protein 4 (MIP-4), macrophage inflammatory protein 5 (MIP-5), LD78β, RANTES, SIS-epsilon (p500), thymus and activation-regulated chemokine (TARC), eotaxin, I-309, human protein HCC-1/NCC-2, human protein HCC-3, and mouse protein C10]. In addition, the therapeutic genes include genes encoding tissue-type plasminogen activator (tPA) or urokinase-type plasminogen activator, and LAL-generating gene to provide sustained thrombolysis for preventing hypercholesterolemia. Further, nucleotide sequences available for treatment of various diseases including cystic fibrosis, adenosine deaminase deficiency, AIDS and other infectious diseases, and malignant and inflammatory diseases are known to be useful as therapeutic genes.

The term "pro-apoptotic gene" as used herein, refers to a nucleotide sequence, the expression of which results in the programmed cell death. Examples of the pro-apoptotic genes include p53, adenovirus E3-11.6K (derived from Ad2 and Ad5) or adenovirus E3-10.5K (derived from Ad), adenovirus E4 gene, Fas ligand, TNF-α, TRAIL, p53 pathway genes and genes encoding a series of caspases.

The term "anti-angiogenic gene" as used herein, refers to a nucleotide sequence, the expression of which results in the extracellular secretion of anti-angiogenic factors. Anti-algiogenesis factors include angiostatin, inhibitors of vascular endothelial growth factor (VEGF) such as Tie 2 (PNAS, 1998, 95, 8795-8800), and endostatin.

The nucleotide sequences of interest described previously are available from DNA sequence databases such as GenBank and EMBL.

The gene delivery system of the present invention is constructed in a variety of forms, preferably, (i) naked recombinant DNA molecule, (ii) plasmid, (iii) viral vector, or (iv) liposome or neosome containing naked recombinant DNA molecule and plasmid.

The relaxin-encoding nucleotide sequence may be applied to a multitude of gene delivery systems useful in gene therapy, preferably, plasmid, adenovirus (Lockett LJ, et al., Clin. Cancer Res. 3:2075-2080(1997)), adeno-associated virus (AAV, Lashford LS., et al., Gene Therapy Technologies, Applications and Regulations Ed. A. Meager, 1999), retrovirus (Gunzburg WH, et al., Retroviral vectors. Gene Therapy Technologies, Applications and Regulations Ed. A. Meager, 1999), lentivirus (Wang G. et al., J. Clin. Invest. 104(11):R55-62(1999)), herpes simplex virus (Chamber R., et al., Proc. Natl. Acad. Sci USA 92:1411-1915(1995)), vaccinia virus (Puhlmann M. et al., Human Gene Therapy 10:649-657(1999)), liposome (Methods in Molecular Biology, Vol 199, S.C. Basu and M. Basu (Eds.), Human Press 2002) or neosome. Most preferably, the gene delivery system of this invention is constructed by incorporating the relaxin-encoding nucleotide sequence to adenoviruses.

### (i) Adenovirus

Adenovirus has been usually employed as a gene delivery system because of its mid-sized genome, ease of manipulation, high titer, wide target-cell range, and high infectivity. Both ends of the viral genome contains 100-200 bp ITRs (inverted terminal repeats), which are cis elements necessary for viral DNA replication and packaging. The E1 region (E1A and E1B) encodes proteins responsible for the regulation of transcription of the viral genome and a few cellular genes. The expression of the E2 region (E2A and E2B) results in the synthesis of the proteins for viral DNA replication.

Of adenoviral vectors developed so far, the replication incompetent adenovirus having the deleted E1 region is usually used. The deleted E3 region in adenoviral vectors may provide an insertion site for transgenes (Thimmappaya, B. et al., Cell, 31:543-551(1982); and Riordan, J. R. et al., Science, 245:1066-1073(1989)). Therefore, it is preferred that the relaxin-encoding nucleotide sequence is inserted into either the deleted E1 region (E1A region and/or E1B region, preferably, E1B region) or the deleted E3 region, more preferably, the deleted E3 region. The nucleotide sequence of interest to be delivered is preferably inserted into either the deleted E1 region (E1A region and/or E1B region, preferably, E1B region) or the deleted E3 region, more preferably, the deleted E1 region. Furthermore, the inserted sequences may be incorporated into the deleted E4 region. The term "deletion" with reference to viral genome encompasses whole deletion and partial deletion as well.

According to the most preferred embodiment, the adenoviral gene delivery system of this invention comprises both "promoter-nucleotide sequence of interest-poly A sequence" and "promoter-relaxin gene-poly A sequence". The promoter-nucleotide sequence of interest-poly A sequence is preferably present in either the deleted E1 region (E1A region and/or E1B region, preferably, E1B region) or the deleted E3 region, more preferably, the deleted E1 region. The promoter-relaxin gene-poly A sequence is preferably present in either'the deleted E1 region (E1A region and/or E1B region, preferably, E1B region) or the deleted E3 region, more preferably, the deleted E3 region. In addition, the adenoviral gene delivery system may comprise a bicistronic expression system in which the nucleotide sequence of interest and relaxin-encoding nucleotide sequence are linked each other by IRES (internal ribosome entry site) to form "promoter-nucleotide sequence of interest-poly A sequence-relaxin gene-poly A sequence.

In nature, adenovirus can package approximately 105% of the wild-type genome, providing capacity for about 2 extra kb of DNA (Ghosh-Choudhury et al., EMBO J., 6:1733-1739(1987)). In this regard, the foreign sequences described above inserted into adenovirus may be further inserted into adenoviral wild-type genome.

The adenovirus may be of any of the 42 different known serotypes or subgroups A-F. Adenovirus type 5 of subgroup C is the most preferred starting material for constructing the adenoviral gene delivery system of this invention. A great deal of biochemical and genetic information about adenovirus type 5 is known.

The foreign genes delivered by the present adenoviral gene delivery system are episomal, and therefore, have low genotoxicity to host cells. Therefore, gene therapy using the adenoviral gene delivery system of this invention may be considerably safe.

### (ii) Retrovirus

Retroviruses capable of carrying relatively large exogenous genes have been used as viral gene delivery vectors in the senses that they integrate their genome into a host genome and have broad host spectrum.

In order to construct a retroviral vector, the relaxin-encoding nucleotide sequences and the nucleotide sequence of interest to be transferred are inserted into the viral genome in the place of certain viral sequences to produce a replication-defective virus. To produce virions, a packaging cell line containing the gag, pol and env genes but without the LTR (long terminal repeat) and ψ components is constructed (Mann et al., Cell, 33:153-159(1983)). When a recombinant plasmid containing the relaxin-encoding sequence, the nucleotide sequence of interest, LTR and ψ is introduced into this cell line, the ψ sequence allows the RNA transcript of the recombinant plasmid to be packaged into viral particles, which are then secreted into the culture media (Nicolas and Rubinstein "Retroviral vectors," In: Vectors: A survey of molecular cloning vectors and their uses, Rodriguez and Denhardt (eds.), Stoneham: Butterworth, 494-513(1988)). The media containing the recombinant retroviruses is then collected, optionally concentrated and used for gene delivery.

A successful gene transfer using the second-generation retroviral vector has been reported. Kasahara et al. (Science, 266:1373-1376(1994)) prepared variants of moloney murine leukemia virus in which the EPO (erythropoietin) sequence is inserted in the place of the envelope region, consequently, pr-oducing chimeric proteins having novel binding properties. Likely, the present gene delivery system can be constructed in accordance with the construction strategies for the second-generation retroviral vector.

### (iii) AAV vector

Adeno-associated viruses are capable of infecting non-dividing cells and various types of cells, making them useful in constructing the gene delivery system of this invention. The detailed descriptions for use and preparation of AAV vector are found in U.S. Pat. No. 5,139,941 and 4,797,368.

Research results for AAV as gene delivery systems are disclosed in LaFace et al, Viology, 162:483486(1988), Zhou et al., Exp. Hematol. (NY), 21:928-933(1993), Walsh et al, J. Clin. Invest., 94:1440-1448(1994) and Flotte et al., Gene Therapy, 2:29-37(1995). Recently, an AAV vector has been approved for Phase I human trials for the treatment of cystic fibrosis.

Typically, a recombinant AAV virus is made by cotransfecting a plasmid containing the gene of interest (i.e., relaxin gene and nucleotide sequence of interest to be delivered) flanked by the two AAV terminal repeats (McLaughlin et al., 1988; Samulski et al., 1989) and an expression plasmid containing the wild type AAV coding sequences without the terminal repeats (McCarty et al., J. Virol., 65:2936-2945(1991)).

### (iv) Other viral vectors

Other viral vectors may be employed as a gene delivery system in the present invention. Vectors derived from viruses such as vaccinia virus (Puhlmann M. et al., Human Gene Therapy 10:649-657(1999); Ridgeway, "Mammalian expression vectors," In: Vectors: A survey of molecular cloning vectors and their uses. Rodriguez and Denhardt, eds. Stoneham: Butterworth, 467-492(1988); Baichwal and Sugden, "Vectors for gene transfer derived from animal DNA viruses: Transient and stable expression of transferred genes," In: Kucherlapati R, ed. Gene transfer. New York: Plenum Press, 117-148(1986) and Coupar et al., Gene, 68:1-10(1988)), lentivirus (Wang G. et al., J. Clin. Invest. 104(11):R55-62(1999)) and herpes simplex virus (Chamber R., et al., Proc. Natl. Acad. Sci USA 92:1411-1415(1995)) may be used in the present delivery systems for transferring both the relaxin gene and nucleotide sequence of interest into cells.

### (v) Liposome

Liposomes are formed spontaneously when phospholipids are suspended in an excess of aqueous medium. Liposome-mediated nucleic acid delivery has been very successful as described in Nicolau and Sene, Biochim. Biophys. Acta, 721:185-190(1982) and Nicolau et al., Methods Enzymol., 149:157-176(1987). Example of commercially accessible reagents for transfecting animal cells using liposomes includes Lipofectamine (Gibco BRL). Liposomes entrapping the relaxin gene and nucleotide sequence of interest interact with cells by mechanism such as endocytosis, adsorption and fusion and then transfer the sequences into cells.

In another aspect of this invention, there is provided a method for delivery a gene, which comprises contacting the gene delivery system of this invention as described hereinabove to a biosample containing cells.

Where the present gene delivery system is constructed on the basis of viral vector construction, the contacting is performed as conventional infection methods known in the art. The infection of hosts using viral vectors is well described in the above-cited publications.

Where the present gene delivery system is a naked recombinant DNA molecule or plasmid, the relaxin-encoding sequence and nucleotide sequence to be delivered are introduced into cells by microinjection (Capecchi, M.R., Cell, 22:479(1980) and Harland and Weintraub, J. Cell Biol. 101:1094-1099(1985)), calcium phosphate co-precipitation (Graham, F.L. et al., Virology, 52:456(1973) and Chen and Okayama, Mol. Cell. Biol. 7:2745-2752(1987)), electroporation (Neumann, E. et al., EMBO J., 1:841(1982) and Tur-Kaspa et al., Mol. Cell Biol., 6:716-718(1986)), liposome-mediated transfection (Wong, T.K. et al., Gene, 10:87(1980) and Nicolau and Sene, Biochim. Biophys. Acta, 721:185-190(1982); and Nicolau et al., Methods Enzymol., 149:157-176(1987)), DEAE-dextran treatment (Gopal, Mol. Cell Biol., 5:1188-1190(1985)), and particle bombardment (Yang et al., Proc. Natl. Acad. Sci., 87:9568-9572(1990)).

In still another aspect of this invention, there is provided a recombinant adenovirus, which comprises an adenoviral ITR (inverted terminal repeat) nucleotide sequence and a relaxin-encoding nucleotide sequence; wherein a relaxin protein expressed enhances a penetration potency of the recombinant adenovirus into a tumor tissue and apoptosis of a tumor cell infected with the recombinant adenovirus.

In the recombinant adenovirus of this invention, the relaxin protein expressed increases significantly a penetration potency of the recombinant adenovirus into a tumor tissue and apoptosis of a tumor cell infected with the recombinant adenovirus, making the therapeutic efficacy of the adenovirus considerably increased.

A small portion of adenoviral genome is known to be necessary as cis elements (Tooza, J. Molecular biology of DNA Tumor viruses, 2nd ed. Cold Spring Harbor Laboratory, Cold Spring Harbor, N.Y.(1981)), allowing substitution of large pieces of adenoviral DNA with foreign sequences, particularly together with the use of suitable cell lines such as 293. In this context, the recombinant adenovirus comprises the adenoviral ITR sequence as an essential sequence as well as the relaxin-encoding nucleotide sequence.

It is preferred that the relaxin-encoding nucleotide sequence is inserted into either the deleted E1 region (E1A region and/or E1B region, preferably, E1B region) or the deleted E3 region, more preferably, the deleted E3 region. The nucleotide sequence of interest to be delivered (e.g., cytokine genes, immuno-costimulatory factor genes, apoptotic genes and tumor suppressor genes) is inserted into the recombinant adenovirus, preferably into either the deleted E1 region (E1A region and/or E1B region, preferably, E1B region) or the deleted E3 region, more preferably, the deleted E1 region (E1A region and/or E1B region, most preferably, E1B region) Furthermore, the inserted sequences may be incorporated into the deleted E4 region.

In nature, adenovirus can package approximately 105% of the wild-type genome, providing capacity for about 2 extra kb of DNA (Ghosh-Choudhury et al., EMBO J., 6:1733-1739(1987)). In this regard, the foreign sequences described above inserted into adenovirus may be further inserted into adenoviral wild-type genome.

According to a preferred embodiment, the recombinant adenovirus of this invention comprises the inactivated E1B 19 gene, inactivated E1B 55 gene or inactivated E1B 19/E1B 55 gene. The term "inactivation" in conjunction with genes used herein refers to conditions to render transcription and/or translation of genes to occur non-functionally, thereby the correct function of proteins encoded genes cannot be elicited. For example, the inactivated E1B 19 gene is a gene incapable of producing the functional E1B 19 kDa protein by mutation (substitution, addition, and partial and whole deletion). The defect E1B 19 gives rise to the increase in apoptotic incidence and the defect E1B 55 makes a recombinant adenovirus tumor-specific (see Korean Pat. Appln. No. 10-2002-0023760).

According to a preferred embodiment, the recombinant adenovirus of this invention comprises the active E1A gene. The adenovirus carrying the active E1A gene is replication-competent. More preferably, the recombinant adenovirus of this invention comprises the inactivated E1B 19/E1B 55 gene and active E1A gene. Most preferably, the recombinant adenovirus comprises the inactivated E1B 19/E1B 55 gene, the active E1A gene and the relaxin-encoding sequence in place of deleted E3 region.

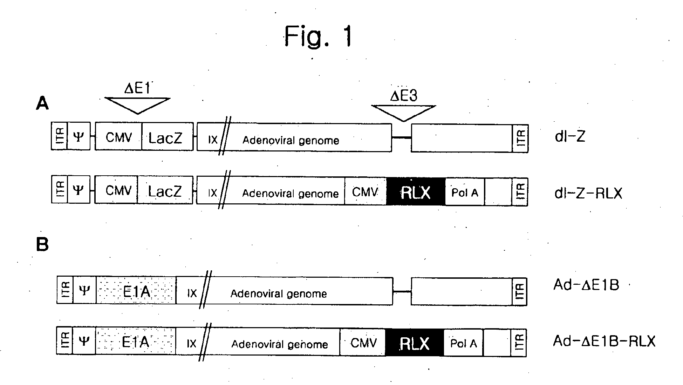

According to the most preferred embodiment, the recombinant adenovirus of this invention comprises a structure of "ITR-E1A-ΔE1B-promoter-relaxin gene-poly A sequence" in which the promoter-relaxin gene-poly A sequence is present in the deleted E3 region. The exemplified adenovirus of this invention has a genetic map represented by Ad-ΔE1B-RLX in Fig. 1.

The recombinant adenovirus of this invention shows highly improved transduction (penetration) efficiency into tumors compared to conventional anti-tumoric adenoviruses and apoptosis potency as well. These improved efficacies are ascribed mainly to relaxin to effectively degrade extracellular matrix and increase apoptotic potential. Consequently, the recombinant adenovirus of this invention exhibits dramatically enhanced oncolytic effect.

Tumor tissues are not agglomerates composed solely of tumor cells but complicated structure further comprising blood vessel and normal cells. In particular, the connective tissue in tumor tissues is generally rigid and forms tight extracellular matrix surrounding tumor cells. Therefore, anticancer drugs as well as viruses cannot penetrate effectively into tumors, so that they generally exhibit a limited anti-tumor effect. Such obstacles can be overcome using the recombinant adenovirus of this invention containing the relaxin gene.

As demonstrated in Examples described hereunder, the adenovirus of this invention with the inserted relaxin gene actively spreads even into the center of tumor spheroids as well as their surface. For in vivo tumor tissues, the relaxin-expressing adenovirus of this invention spreads widely and remotely to the distal site from injection site (needle track). The improvement in the transduction efficiency accomplished by the relaxin-expressing adenovirus is obvious even to be easily differentiated with naked eyes. It could be appreciated that the improved transduction efficiency is very considerable compared to about 2-3 fold increase in transduction efficiency of pretreatment of proteases such as collagenase/dispase or trypsin, elastase to degrade elasitin or hyaluronidase to degrade extracellular matrix.

The enhanced spreading effect within tissues by relaxin can greatly increase anti-tumor efficacy of tumor-specific oncolytic adenovirus. This improved anti-tumor efficacy may be exhibited in replication incompetent adenoviruses as well as replication competent adenoviruses. The enhanced ability of adenoviruses to induce apoptosis by relaxin is surprising and non-anticipated.

In further aspect of this invention, there is provided a pharmaceutical anti-tumor composition for treating a cancer, which comprises (a) a therapeutically effective amount of the recombinant adenovirus described previously; and (b) a pharmaceutically acceptable carrier.

In still further aspect of this invention, there is provided a method for treating a cancer, which comprises administering to an animal the pharmaceutical anti-tumor composition of described above.

The recombinant adenovirus as an active ingredient in the pharmaceutical composition is the adenovirus of the present invention described hereinabove and therefore the above descriptions can be adapted to the recombinant adenovirus of the pharmaceutical composition. Accordingly, the common descriptions between them are omitted in order to avoid undue redundancy leading to the complexity of this specification.

To effectively elicit anti-tumor effect by recombinant adenoviruses, it is necessary that viruses proliferate and spread to neighboring cells faster than the growth rate of cancer cells to induce oncolytic effect. In addition, a successful cancer-gene therapy using adenoviruses requires enhanced safety as well as high therapeutic benefit. The relaxin-expressing adenovirus of this invention increases both viral spreading and apoptosis to exhibit significantly increased anti-tumor effect. In particular, the recombinant adenovirus of this invention having deleted E1B 55 gene shows excellent tumor-specificity in cytotoxicity. For this reason, the relaxin-expressing adenovirus of this invention allows to decrease a dosage for cancer therapy, reducing significantly toxicity to normal cells and undesirable immune reactions *in vivo*

Since the recombinant adenovirus of this invention has oncolytic effect to a wide variety of tumor cells, the pharmaceutical composition of this invention is useful in treating tumor-related diseases, including stomach cancer, lung cancer, breast cancer, ovarian cancer, liver cancer, bronchogenic cancer, nasopharyngeal cancer, laryngeal cancer, pancreatic cancer, bladder cancer, colon cancer, and uterine cervical cancer. The term "treatment" as used herein, refers to (i) prevention of tumorigenesis; (ii) suppression and curing of tumor-related diseases or disorders by eradicating tumor cells; and (iii) alleviation of tumor-related diseases or disorders by eradicating tumor cells. Therefore, the term "therapeutically effective amount" as used herein means an amount sufficient to achieve the pharmaceutical effect described above.

The pharmaceutically acceptable carrier contained in the pharmaceutical composition of the present invention, which is commonly used in pharmaceutical formulations, but is not limited to, includes lactose, dextrose, sucrose, sorbitol, mannitol, starch, rubber arable, potassium phosphate, arginate, gelatin, potassium silicate, microcrystalline cellulose, polyvinylpyrrolidone, cellulose, water, syrups, methyl cellulose, methylhydroxy benzoate, propylhydroxy benzoate, talc, magnesium stearate, and mineral oils. The pharmaceutical composition according to the present invention may further include a lubricant, a humectant, a sweetener, a flavoring agent, an emulsifier, a suspending agent, and a preservative.

The pharmaceutical composition according to the present invention may be administered via the routes used commonly in gene therapy, and preferably, administered parenterally, i.e., by intravenous, intraperitoneal, intramuscular, subcutaneous, or local administration. For example, the pharmaceutical composition may be administered intraperitoneally to treat ovarian cancer and intravenously to treat liver cancer, directly injected to visible tumor mass to treat breast cancer, directly injected to enema to treat colon cancer, and directly injected to a catheter to treat bladder cancer.

A suitable dosage amount of the pharmaceutical composition of the present invention may vary depending on pharmaceutical formulation methods, administration methods, the patient's age, body weight, sex, pathogenic state, diet, administration time, administration route, an excretion rate and sensitivity for a used pharmaceutical composition, and physicians of ordinary skill in the art can determine an effective amount of the pharmaceutical composition for desired treatment. Generally, the pharmaceutical composition of the present invention comprises 1 x 10⁵ - 1 x 10¹⁵ pfu/ml of a recombinant adenovirus, and 1 x 10¹⁰ pfu of a recombinant adenovirus is typically injected once every other day over two weeks.

According to the conventional techniques known to those skilled in the art, the pharmaceutical composition comprising the recombinant adenovirus according to the present invention may be formulated with pharmaceutically acceptable carrier and/or vehicle as described above, finally providing several forms a unit dose form and a multi-dose form. Non-limiting examples of the formulations include, but not limited to, a solution, a suspension or an emulsion in oil or aqueous medium, an extract, an elixir, a powder, a granule, a tablet and a capsule, and may further comprise a dispersion agent or a stabilizer.

The pharmaceutical composition comprising the recombinant adenovirus according to the present invention may be utilized alone or in combination with typical chemotherapy or radiotherapy. Such combination therapy may be more effective in treating cancer. The chemotherapeutic agents useful for the combination therapy include cisplatin, carboplatin, procarbazine, mechlorethamine, cyclophosphamide, ifosfamide, melphalan, chlorambucil, bisulfan, nikosourea, dactinomycin, daunorubicin, doxorubicin, bleomycin, plicomycin, mitomycin, etoposide, tamoxifen, taxol, transplatinum, 5-fluorouracil, vincristin, vinblastin and methotrexate. Examples of the radiotherapy useful for the combination therapy include X-ray illumination and γ-ray illumination.

In another aspect of this invention, there is provided a pharmaceutical composition for improving a penetration potency of a medicament into a tissue, which comprises (a) a relaxin protein to improve the penetration potency of the pharmaceutical composition into the tissue; and (b) a pharmaceutically acceptable carrier.

The relaxin protein contained the pharmaceutical composition of this invention may be obtained from natural sources and conventional DNA recombinant technologies. Furthermore, its fragments are encompassed in the present invention unless they are inactive in the degradation of extracellular matrix.

The pharmaceutical composition may be administered prior to or simultaneously with administration of certain medicament. In addition, the pharmaceutical composition may further comprise a medicament. The pharmaceutical composition of this invention degrades extacellular matrix surrounding tissues to be targeted by medicaments to enhance tissue penetration of medicaments, increasing significantly a pharmacological efficacy of medicaments.

The pharmaceutical acceptable carrier, administration route and method, and formulation for the present pharmaceutical composition are described with referring to descriptions for the pharmaceutical anti-tumor composition of this invention as discussed previously. In particular, the present pharmaceutical composition is preferably administered parenterally, e.g., by intravenous, intraperitoneal, intramuscular, subcutaneous or transdermal and local (e.g., direct injection into brain or breast tumor mass) administration. Generally, the pharmaceutical composition of this invention may be administered in a dosage of 0.0001-100 mg/kg.

The medicament to show improved tissue penetration by the pharmaceutical composition of this invention includes chemical drugs and biodrugs, preferably, drugs whose tissue penetration is deteriorated by extracellular matrix, e.g., anticancer drugs.

In still another aspect of this invention, there is provided a pharmaceutical composition for treating a disease or condition associated with accumulation of excess extracelluar matrix, which comprises (a) a therapeutically effective amount of a relaxin protein or a gene delivery system comprising a relaxin-encoding nucleotide sequence; and (b) a pharmaceutically acceptable carrier.

The pharmaceutical composition of this invention degrades effectively extracellular matrix surrounding tissues to have a therapeutic efficacy on a disease or condition associated with accumulation or deposition of excess extracellular matrix. The phrase "accumulation of excess extracellular matrix" means excessive deposition of components of extracellular matrix such as collagen, laminin, fibronectin and proteoglycan to damage tissues or organs, finally causing fibrosis.

The diseases or conditions associated with excessive accumulation of extracellular matrix to be treated by the present pharmaceutical composition are fibrosis-related diseases, including, , but not limited to, scar, liver cirrhosis, pulmonary fibrosis, glomerular nephritis, adult or acute dyspnea, hepatic fibrosis, renal fibrosis, mycocardial fibrogenesis following myocardial infarction, fibrocystic disorder, fibrotic cancer, veno-occlusive syndrome and renal stroma fibrosis.

Both scar caused by wound, burn or operation and excessive scar such as keloid may be treated with the pharmaceutical composition of this invention.

The gene delivery system comprising a relaxin-encoding nucleotide sequence can be described with referring to descriptions of the gene delivery system of this invention discussed hereinabove. The relaxin protein contained the pharmaceutical composition of this invention may be obtained from natural sources and conventional DNA recombinant technologies. Furthermore, its fragments are encompassed in the present invention unless they are inactive in the degradation of extracellular matrix.

The pharmaceutical acceptable carrier, administration route and method, and formulation for the present pharmaceutical composition are described with referring to descriptions for the pharmaceutical anti-tumor composition of this invention as discussed previously. In particular, the present pharmaceutical composition is most preferably administered by transdermal administration. The formulations suitable in the present pharmaceutical composition include ointment, gel, cream, solution, spray, patch and lotion. Generally, the pharmaceutical composition of this invention may be administered in a dosage of 0.0001-100 mg/kg.

The present invention provides a novel gene delivery system and recombinant adenovirus comprising the relaxin-encoding sequence, a gene delivering method using the gene delivery system, a pharmaceutical anti-tumor composition comprising the recombinant adenovirus, a pharmaceutical composition characterized by improved tissue penetration potency and a pharmaceutical composition for treating a disease or disorder associated with accumulation of excess extracellular matrix. According to the present invention, relaxin is responsible for the improvement in transduction efficacy and apoptotic ability to increase tumor cell killing potential dramatically.

### BRIEF DESCRIPTION OF THE DRAWINGS

Fig. 1 schematically represents a genetic map of the recombinant adenoviruses used in Examples.
Fig. 2 is a graph showing relaxin expression profiles of the recombinant adenovirus of this invention.
Fig. 3 is a photograph representing *in vitro* tissue penetration of the relaxin-expression adenovirus of this invention into tumors such as U343, U87MG, C33A and A549.
Fig. 4 is a photograph representing *in vivo* tissue penetration of the relaxin-expression adenovirus of this invention into tumors such as U343, U87MG, C33A, Hep3B and A549.
Fig. 5 represents the results of CPE (cytopathic effect) analysis demonstrating cell killing potency of the relaxin-expressing adenovirus of this invention.
Fig. 6 represents the results of plaque development assay of the relaxin-expressing adenovirus of this invention.
Fig. 7 shows the results of flow cytometry analysis for subG₁ cell population verifying the ability of the recombinant adenovirus of this invention to induce apoptosis.
Fig. 8 shows the results of flow cytometry analysis for Annexin-V and PI dual staining verifying the ability of the recombinant adenovirus of this invention to induce apoptosis.
Fig. 9 represents the results of TUNEL assay for DNA fragmentations demonstrating the ability of the recombinant adenovirus of this invention to induce apoptosis.
Fig. 10 represents *in vivo* anti-tumor effect of the relaxin-expressing adenovirus of this invention
Fig. 11 represents the increase in survival rate of tumor-bearing mice injected with the relaxin-expressing adenovirus of this invention.
Fig. 12 represents histological changes of tumors in tumor-bearing mice injected with the relaxin-expressing adenovirus of this invention.
Fig. 13 represents collagen distribution within tumors in tumor-bearing mice injected with the relaxin-expressing adenovirus of this invention.

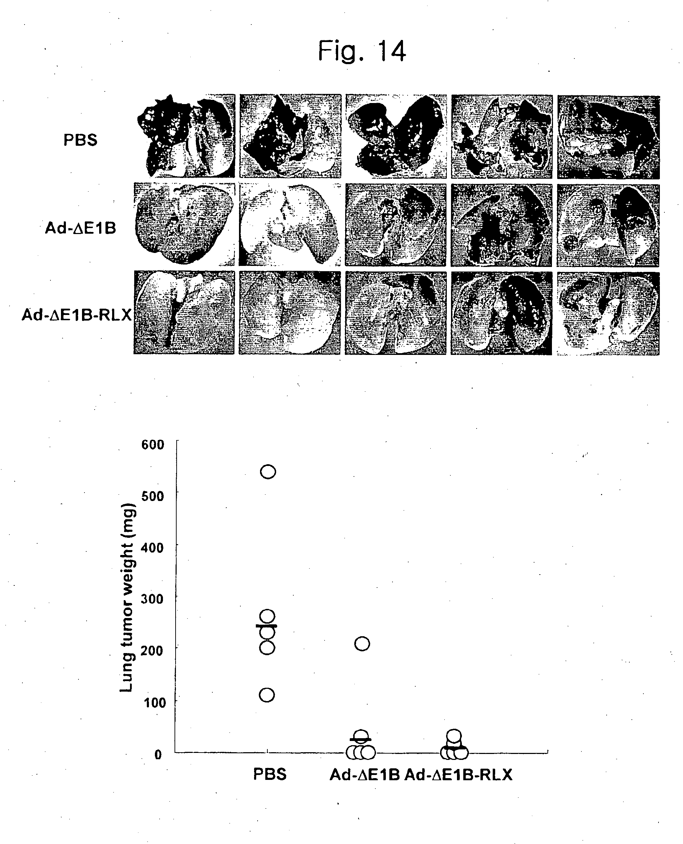

Fig. 14 represents inhibitory effect of the recombinant adenovirus of this invention on spontaneous pulmonary metastasis. B16BL6 tumor-bearing mice were treated with PBS, Ad-ΔE1B, or Ad-ΔE1B-RLX three times every other day, and then the primary tumors were surgically removed. On day 25 after primary tumor removal, the weight of metastatic lesions in the lungs of the mice was assessed. Each point represents the tumor burden for every individual mouse (5 mice per group) and the mean weight of metastatic lesions for each group is shown with a line. **P* < 0.01 vs PBS-treated control and **P* < 0.05 vs Ad-ΔE1B-treated group.
Fig. 15 is a photograph demonstrating the transduction efficiency and penetration potency of the relaxin-expressing adenovirus of this invention to keloid cells, cell spheroids and tissue spheroids.

The following specific examples are intended to be illustrative of the invention and should not be construed as limiting the scope of the invention as defined by appended claims.

### EXAMPLES

### MATERIALS and METHODS

### Cell lines and cell culture

Cell lines for experiments were human brain cancer cell lines (U343, U87MG), cervical cancer cell line (C33A), liver cancer cell line (Hep3B), lung cancer cell line (A549) and 293 cell line carrying the early gene of adenovirus, E1 region available from the American Type Culture Collection (ATCC). All cell lines were cultured in Dulbecco's modified Eagle's medium (DMEM; Gibco BRL) supplemented with 10% fetal bovine serum (Gibco BRL), penicillin and streptomycin and maintained at 37°C under 5% CO₂ atmosphere.

### Generation and titration of recombinant adenoviruses

To generate El/E3-gene deleted replication-incompetent adenoviruses expressing relaxin and lac Z as a reporter, we first constructed pdl-LacZ viral vector with lac Z gene at the deleted E1 region using vmd1324Bst (gifted from Dr. Verca, University of Fribourgh, Switzerland; Heider, H. et al., Biotechniques, 28(2):260-265, 268-270(2000). For preparing this vector, the pcDNA-hygro-LacZ plasmid (Invitrogen, Carlsbad, CA, USA) was digested with *Hind*III and *Nae*I to isolate the CMV promoter, lacZ gene and polA and the isolated three sequences were inserted into the E1 adenoviral shuttle vector, pΔE1sp1A to prepare pΔE1sp1A/CMV-LacZ shuttle vector. The prepared pΔE1sp1A/CMV-LacZ shuttle vector was digested with *Xmn*I and cotransformed with vmd1329Bst adenovirus linearized by BstBI into *E*. *coli* BJ5183 (Dr. Verca, University of Fribourgh, Switzerland) to induce homologous recombination, obtaining pdl-LacZ adenovirus.

For constructing relaxin-expressing adenoviruses, pDNR-LIB-RLX (ATCC, #MGC-14599) was digested with *Sal*I-*Hind*III to obtain a 1 kb DNA fragment which in turn was subcloned into the pCA14 vector (Microbix, Ontario, Canada), generating a pCA14-RLX. The nucleotide sequences of relaxin used in the Example is published under GenBank accession No. BC005956. Then, a CMV-RLX-polA expression cassette was excised from the pCA14-RLX using *Bgl*II and subsequently inserted into the adenovirus E3 shuttle vector pSP72ΔE3 (Promega, Madison, WI, USA) to construct a pSP72ΔE3-cRLX E3 shuttle vector. The constructed pSP72ΔE3-cRLX E3 shuttle vector was linearized with *Xmn*I and then cotransformed with the pdl-LacZ into *E. coli* BJ5183 (Dr. Verca, University of Fribourgh, Switzerland) to induce homologous recombination, producing a dl-LacZ-RLX (or dl-Z-RLX) adenovirus vector (Fig. 1). The dl-Z-RLX adenovirus was deposited in the Korean Culture Center of Microorganisms (KCCM) with the Accession No. KCCM-10567 on March 19, 2004.

To construct a replication-competent adenovirus expressing the relaxin gene, the pSP72ΔE3-cRLX E3 shuttle vector prepared above was linearized with *Xmn*I and cotransformed into *E*. *coli* BJ5183 together with the E1B 19 kDa/E1B 55 kDa-deleted pAdΔE1B19/55 adenovirus vector linearized with *Spe*I (KFCC 11288) for homologous recombination, generating a Ad-ΔE1B-RLX adenovirus vector (Fig. 1). The Ad-ΔE1B-RLX adenovirus was deposited in the Korean Culture Center of Microorganisms (KCCM) with the Accession No. KCCM-10566 on March 19, 2004.

In Fig. 1, ψ denotes a sequence comprising ITR (inverted terminal repeat) and the package signal, Ad represents adenovirus, CMV represents the CMV promoter, Pol A is the poly A sequence and IX represents a gene encoding the IX protein.

To verify the respective homologous recombinants, the plasmid DNA was digested with *Hind*III and the digestion pattern was analyzed. The proper homologous recombinant adenoviral plasmid DNA was digested with *Pac*I and transfected into 293 cells to generate dl-lacZ-RLX and Ad-ΔEIB-RLX adenoviruses. All viruses were propagated in 293 cells and their titration was performed according to limited dilution or plaque assay (Hitt, M. et. al., Construction and propagation of human adenovirus vectors. Cell biology: a laboratory handbook. New York: Academic Press Inc, 479-490(1994)), followed by concentration using CsCl gradient and purification.

As a control virus, an Ad-ΔEIB (with deleted E1B region) was constructed and produced using the pCA14 as E1 shuttle vector according to procedures described previously.

### Examination of relaxin expression pattern in dI-LacZ-RLX and Ad-ΔE1B19/55-RLX

Human cervical cancer cell line C33A was infected with dl-LacZ-RLX or Ad-ΔE1B-RLX, or dl-LacZ or Ad-ΔE1B19 adenovirus (KFCC-11288) at MOI (multiplicity of infection) of 1-50 and medium used was recovered after 24 hr. The expression of relaxin was analyzed using the ELISA kit (Immune diognostik, Benshem, Germany) according to the manufacturer's protocol.

### Evaluation on spreading and penetration of dI-LacZ-RLX in tumor spheroid

U343, U87MG, C33A and A549 xenografts were established subcutaneously by injecting cells into the abdomen of 6- to 8-week-old nude mice and once the tumors reached to 150-200 mm³ in volume, fresh tumor tissue was extracted at surgery. 1-2 mm fragments of the tumor tissue were dissected. These explants were plated individually on 0.75% agarose-coated plates and cultured in DMEM (Gibco BRL) supplemented with 5% FBS (Gibco BRL) and penicillin/streptomycin (Gibco BRL) at 37°C under 5% CO₂ atmosphere. Medium was renewed once every week. Prior to infection with adenoviruses, spheroids with diameter of 2 mm were transferred to 0.75% agarose-coated 48-well plates and 150 µl of DMEM (containing 5% FBS) were added, after which viruses were added at 1x10⁶, 1x 10⁷, or 1 x 10⁸ PFU. 48-hr later, the medium was aspirated and spheroids were fixed in a fixation solution for X-gal staining. The surface of X-gal stained spheroids was observed under a stereoscopic microscope. For the observation on penetration of adenovirus into tumor spheroid, the X-gal stained tumor spheroids were embedded in O.C.T. compound (Sakura Finetec, Torrance, CA) and snap frozen. 8 µm frozen section was then placed onto gelatin-coated slide glass.

### Evaluation on spreading and penetration of dI-LacZ-RLX in vivo

U343, U87MG, C33A, Hep3B and A549 xenografts were established subcutaneously by injecting cells into the abdomen of 6- to 8-week-old nude mice and once the tumors reached to 150-200 mm³ in volume, mice were randomized into two groups and dI-LacZ-RLX adenovirus at 5 × 10⁷ - 1 × 10⁸ PFU in 50 µl was intratumorally injected into the tumors three times. Three days after the last injection, animals were sacrificed and tumors were taken for virus distribution, after which they were fixed in 4% paraformaldehyde at 4°C for 4-8 hr and dehydrated in 30% sucrose solution for 12 hr. The dehydrated tumor tissues were frozen in O.C.T. compound and dissected to sections with 8 µm thickness, followed by placing them onto gelatin-coated slide glass for X-gal staining.

### Cytopathic effect (CPE) of Ad-ΔE1B-RLX virus

To evaluate the oncolytic activity of relaxin-expressing adenoviruses, human tumor cell lines (U343, U87MG, C33A, Hep3B and A549) were plated onto 24-well plates and then infected with Ad-ΔE1, Ad-ΔE1B, or Ad-ΔE1B-RLX adenovirus at MOIs 0.1-10. At the time that cells infected with any one of the viruses exhibited complete cell lysis at an MOI of 0.1-0.5, the dead cells were washed out and cells on the plate were then stained with 0.5% crystal violet in 50% methanol.

### Plaque development assay

To observe the change of plaque size over relaxin expression, 3 x 10⁵ Hep3B cells were placed to 6-well plates and infected with Ad-ΔE1B or Ad-ΔE1B-RLX adenovirus at 3 MOI after one day of cell growth. After 4 hr of incubation, the infected cells were overlayed with agarose-DMEM mixture of 2 x DMEM (containing 10% FBS and penicillin/streptomycin) at 37°C and 1.4% agarose at 42°C and then incubated at 37°C in 5% CO₂ incubator. Following about 10 days of incubation, agarose overlay was removed after soaking with 10% TCA (trichoroacetic acid) for 30 minutes and the remaining cells were stained with 0.5% crystal violet in 50% methanol.

### Flow cytometry analysis for apoptosis potential

To examine apoptosis induced by relaxin, human tumor cell lines, U343, U87MG, C33A, Hep3B and A549, were introduced to 25T culture flasks and 24 hr later, infected with Ad-ΔE1B or Ad-ΔE1B-RLX adenovirus at an MOI of 0.5-5. Cells were treated with 0.1-1 µM CPT-11 (camptothecin) as a positive control and treated with PBS as a negative control. After 48 hr, 72 hr and 96 hr of infection, the infected cells were collected and fixed in 70% ethanol at 4°C for 24 hr. Following the fixation, the cells were incubated with a mixture of PI (propidium iodide, 50 µg/µl) and RNase for 15 min and the analysis by flow cytometry was performed. In addition, to examine early apoptosis induced by relaxin, several human tumor cell lines were infected with Ad-ΔE1B or Ad-ΔE1B-RLX adenovirus as described above. The infected cells were collected and then processed for Annexin V/PI dual staining according to manufacturer's instruction in the ApoAlert V-FITC apoptosis kit (Clontech, Palo Alto, CA), followed by flow cytometric analysis.

### TUNEL assay

U343 (5 x 10⁴), U87MG (5 x 10⁴) C33A (5 x 10⁵), Hep3B (4 x 10⁵) , and A549 (5 x 10⁴) cells were plated onto a chamber slide and then infected with adenovirus at an MOI of 0.2-20. Following 24 hr and 48 hr of infection, medium was removed and TUNNEL assay was carried out according to the manufacturer's instruction of ApopTag kit (Intergen, 'Purchase, NY). For color development, cells were incubated with peroxidase-conjugated streptavidin and then diaminobenzidine (DAKO, Carpinteria, CA). At the time that color of cells became brown, cells were counterstained with 0.5% methyl green for 10 min and observed under microscope in more than 4 selected fields. The ratio of stained cells to total cells was calculated.

### Anti-tumor effects of relaxin-expressing adenovirus in vivo

To assess the effect of Ad-ΔE1B-RLX adenovirus on the growth of human tumor spheroid formed in nude mice, tumors were implanted on the abdomen of 6- to 8-week-old nude mice by subcutaneous injection of 1 x 10⁷ cancer cells (U343, U87MG, C33A, Hep3B and A549). When tumors reached to a range of 50-80 mm³, Ad-ΔE1B or Ad-ΔE1B-RLX at 5 x 10⁷ - 5 x 10⁸ PFU in PBS was administered intratumorally three times every other day and the growth pattern of tumors was observed. The volume of tumors was calculated with the major axis and minor axis measured using a caliper: volume = (minor axis mm)² x (major axis mm) x 0.523.

### Observation of the change of tumor characteristics induced by the administration of relaxin-expressing replication-competent adenovirus

When C33A tumor formed in the abdomen of nude mice reached to about a range of 50-80 mm³, Ad-ΔE1B or Ad-ΔE1B-RLX at 5 x 10⁷ PFU in PBS was administered intratumorally three times. Following 3 days of injection, the tumor tissues were extracted and their paraffin blocks were prepared. The blocks were cut into 4-µm slides and deparaffinized in xylene and then in graded alcohols (100%, 95%, 80% and 70%), followed by staining with hematoxylin and eosin. For the observation of distribution of collagen, a component of connective tissue, 4-µm paraffin-embedded slides were stained using boulin, hematoxylin and biebrich's scarlet acid fuchsin.

In addition, the immunohistochemistry staining for the hexon region of adenoviruses was carried out. The slides were deparaffinized as described above and incubated with the primary anti-adenoviral hexon antibody, AB1056F (Chemicon, Temecula, CA) and then with the secondary goat anti-rat IgG-HRP (Santa Cruz Biotechnology, Inc., Santa Cruz, CA). The color development was performed using DAB (DAKO, Carpinteria, CA).

To observe the occurrence of apoptosis in tumors, TUNNEL assay was carried out according to the manufacturer's instruction of ApopTag kit (Intergen, Purchase, NY). For the color development, cells were incubated with peroxidase-conjugated streptavidin and then diaminobenzidine (DAKO, Carpinteria, CA). At the time that color of cells became brown, cells were counterstained with 0.5% methyl green for 10 min and observed under microscope.

### Murine B16BL6 spontaneous lung metastasis model

Spontaneous metastasis model was used to examine the effect of relaxin gene administration on tumor metastasis. More specifically, B16BL6 cells (2 x 10⁵/mouse) were administered subcutaneously into the right hind foot pad of 6-week-old male C57BL/6 mice (Charles River Korea, Seoul, Korea). Once the tumor volume reached to a volume of 100-200 mm³, animals were randomized into three groups (PBS, Ad-ΔE1B, Ad-ΔE1B-RLX) of 5 animals each, and treatment was initiated. First day of treatment was designated as day 1, and adenoviruses or PBS were injected directly into the tumor (5 x 10⁸ PFU per tumor in 50 µl of PBS) on days 1, 3, and 5. On day 7, the primary tumors were surgically removed by amputating below knee under mild anesthesia. On day 25 following primary tumor removal, the weight of metastatic tumor lesions in the lungs of the mice was assessed.

### Evaluation on transduction efficiency to keloid cell, cell spheroid and tissue spheroid

To assess the therapeutic efficacy of relaxin-expressing adenoviruses on keloid, we examined the gene transduction efficiency to keloid cell, cell spheroid and tissue spheroid and penetration efficiency of viruses into tissues.

The gene transduction efficiency to keloid cells was assessed as follows: The primary keloid cell line at passage 2 obtained from keliod patients was transferred to 24-well plates and then infected with Ad-ΔE1B or Ad-ΔE1B-RLX virus at MOI of 10 or 50, followed by X-gal staining after 2 days of virus infection.

The gene transduction efficiency to keloid cell spheroid was assessed as follows: The keloid tissues were extracted from keliod patients. 1 x 10⁵/ml of primary cells at passage 2 obtained from the extracted keloid tissues were transferred to a culture tube and centrifuged at 500 x g for 5 min, after which they were cultured at 37°C. When the round pellet detached from the bottom of the tube appeared as a cell spheroid over 1-5 days of culture, the spheroid was transferred to 0.75% agarose-coated 48-well plate and 150 µl of DMEM (containing 5% FBS) were added, after which adenoviruses at 1 x 10⁷ PFU were infected to the spheroid. Following 3 days of virus infection, the keolid spheroid was fixed in a fixation solution and X-gal stained. The surface of X-gal stained spheroids was observed under a stereoscopic microscope. For the observation of penetration of adenoviruses into spheroid, the X-gal stained keloid spheroids were embedded in O.C.T. compound and snap frozen. 10 µm frozen section was then placed onto gelatin-coated slide glass.

The penetration of virus into keloid tissue spheroid was analyzed as follows: The keloid tissues were extracted from keliod patients and dissected to 1-2 mm sections. The sections were cultured in 0.75% agarose-coated incubator in DMEM containing 5% FBS and penicillin/streptomycin. The medium used was refreshed once or twice every week and the keloid tissues were cultured for more than one week. Keloid tissue spheroids in a diameter of 2 mm were transferred to 0.75% agarose-coated 48-well plate and 150 µl of DMEM (containing 5% FBS) were added, followed by the infection with 1 x 10⁶ PFU adenoviruses. Following 3 days of virus infection, the keolid tissue spheroid was fixed in a fixation solution and X-gal stained. The surface of X-gal stained spheroids was observed under a stereoscopic microscope. For the observation of penetration of adenoviruses into spheroid, the X-gal stained keloid spheroids were embedded in O.C.T. compound and snap frozen. 10 µm frozen section was then placed onto gelatin-coated slide glass.

### Statistical Analysis

The data were expressed as mean ± standard error of the mean (SEM). Statistical comparison was made using Stat View software (Abacus Concepts, Inc., Berkeley, CA) and the Mann-Whitney test (nonparametric rank sum test). The criterion for statistical significance was taken as *P* < 0.05.

### RESULTS

### Construction of relaxin-expressing adenoviruses and expression pattern of relaxin

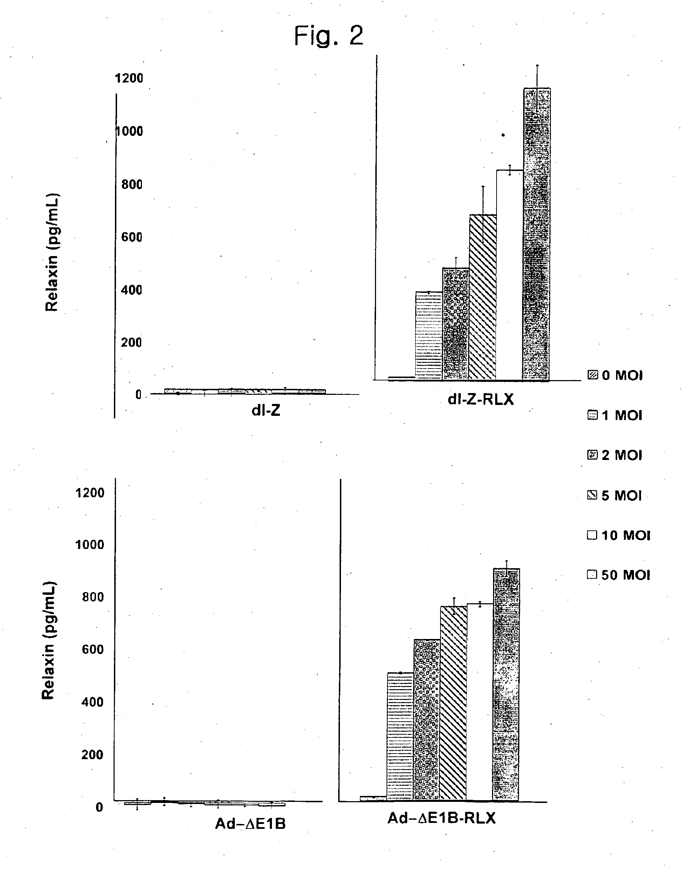

To visually evaluate the alteration of penetration efficiency into tissues depending on relaxin expression, replication-incompetent dl-LacZ-RLX adenoviruses expressing LacZ as a reporter were constructed. Furthermore, tumor-specific oncolytic Ad-ΔE1B-RLX adenovirus was constructed to enhance the transduction efficiency of replication-competent adenovirus into tissues (Fig. 1). For assessing the relaxin expression pattern of adenoviruses constructed, a cervical cancer cell line, C33A was infected with dl-LacZ, dl-LacZ-RLX, Ad-ΔE1B or Ad-ΔE1B-RLX adenoviruses at various MOIs and media were recovered for ELISA (Fig. 2). Cells infected with dl-LacZ as a negative control for replication-incompetent adenovirus and Ad-ΔEIB as a negative control for tumor-specific oncolytic adenovirus were revealed not to express relaxin, whereas those infected with dl-LacZ-RLX and Ad-ΔE1B-RLX showed the dose-dependent expression of relaxin depending on the titer of adenoviruses administered.

### Evaluation on the transduction efficiency of dl-LacZ-RLX adenovirus to in vitro tumor tissue using tumor spheroids

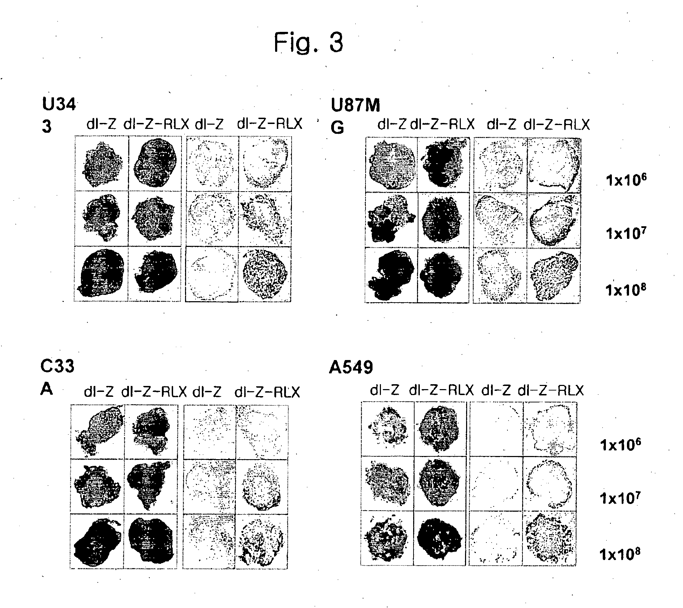

To evaluate the transduction efficiency and tissue penetration of dl-LacZ-RLX to tumor spheroids, various human tumor cell lines were subcutaneously injected into nude mice and once the tumors reached to 150-200 mm³ in volume, fresh tumor tissues was extracted. The tumor tissues extracted were dissected into 1-2 mm fragments and infected with adenoviruses at 1 x 10⁶, 1 x 10⁷, or 1 x 10⁸ PFU. X-gal staining was carried out after 48 hr of infection. Compared to the treatment of 1 x 10⁶ PFU dl-LacZ, the same dose of dl-LacZ-RLX showed stronger X-gal staining on the surface of tumor spheroid. dl-LacZ-RLX at 1 x 10⁷ and 1 x 10⁸ PFU led to darker X-gal staining on the overall surface of tumor spheroid. To accurately investigate the penetration efficiency of adenoviruses into tumor spheroids, X-gal-stained tumor spheroids were sectioned for observation. dl-LacZ at 1 x 10⁶, 1 x 10⁷ or 1 x 10⁸ PFU exhibited poor LacZ expression in tumor tissues and its spread was limited to the surface of tumor spheroids. In contrast, the same doses of dl-LacZ-RLX showed much higher LacZ expression level and its spread was extended to the inner part of tumor spheroids (Fig. 3). The results clearly demonstrate that in the three-dimensional structure of tumor spheroids, relaxin-expressing dl-LacZ-RLX adenovirus transduced and spread to the core of the spheroid with higher efficiency than the control vector, dl-LacZ.

### Evaluation on the transduction efficiency of dl-LacZ-RLX adenovirus in tumor mass in vivo

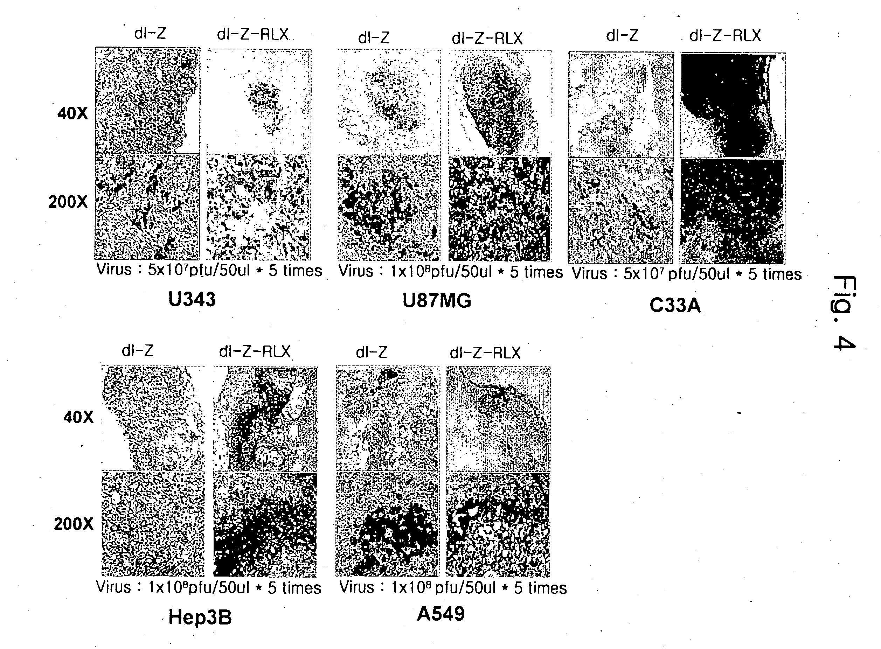

In order to investigate whether the enhanced transduction efficiency and viral spread of dl-LacZ-RLX seen in tumor spheroids in vitro would lead to an increase in lacZ gene delivery to tumor mass in vivo, tumor xenograft models were used. dl-LacZ or dl-LacZ-RLX adenovirus at 5 x 10⁷ or 1 x 10⁸ PFU was intratumorally injected into the tumor mass formed in the abdomen of nude mice. Three days later, tumors were taken and sectioned for X-gal staining. While dl-LacZ exhibited the low level of LacZ expression and the stained region was restricted to the virus injection site, dl-LacZ-RLX showed much higher LacZ expression and the stained region was found to be widely spread to other regions than the virus injection site (Fig. 4). In particular, U87MG and C33A tumor mass transduced with dl-LacZ-RLX showed dark blue color ascribed to intensive LacZ expression throughout all the tumor tissues. These expression profiles address that the penetration and spreading of dl-LacZ-RLX in tumor mass *in vivo* is enhanced compared to dl-LacZ control virus not to express relaxin.

### Assessment on tumor cell killing effect of relaxin-expressing oncolytic adenovirus

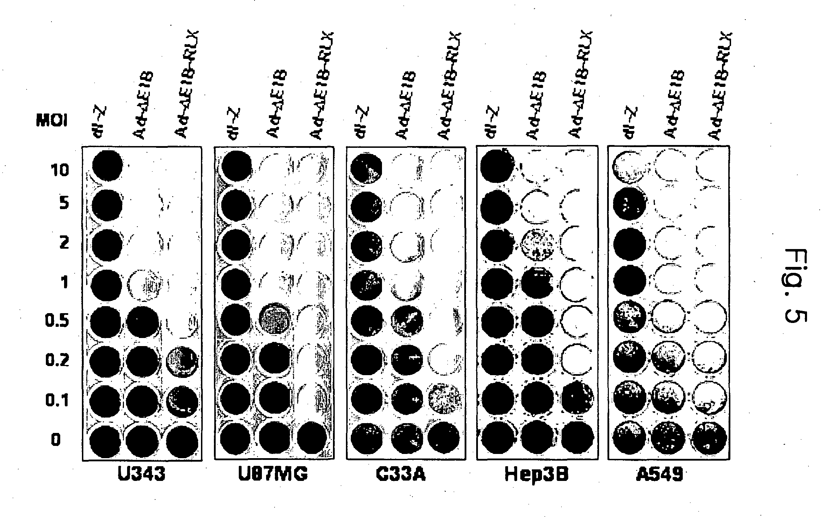

To reveal that the increase in penetration and spreading of relaxin-expressing adenovirus contributes to enhanced tumor cell killing effect of tumor-specific oncolytic adenoviruses, a CPE assay was carried out. Each of human tumor cell lines (U343, U87MG, C33A, Hep3B and A549) was infected with dl-LacZ (negative control), Ad-ΔE1B or Ad-ΔE1B-RLX adenovirus at MOIs 0.1-10 and the resulted tumor cell killing effects were analyzed. As shown in Fig. 5, while the negative control, dl-LacZ elicited little or no cell killing effect in various tumor cell lines, Ad-ΔE1B-RLX exhibited about 2-10 fold higher tumoricidal effect than Ad-ΔE1B not to express relaxin. In particular, Ad-ΔE1B-RLX adenovirus showed about 10-fold higher tumoricidal effect than Ad-ΔE1B in Hep3B cell line, and Ad-ΔE1B-RLX showed about 5-fold higher tumoricidal effect than Ad-ΔE1B in U87MG, C33A and A549 cell lines. According to the results, it could be understood that the relaxin expression does not deteriorate a replication competency of adenoviruses and contributes to the dramatic increase in tumoricidal effect of adenoviruses.

### Plaque formation of relaxin expressing oncolytic adenovirus

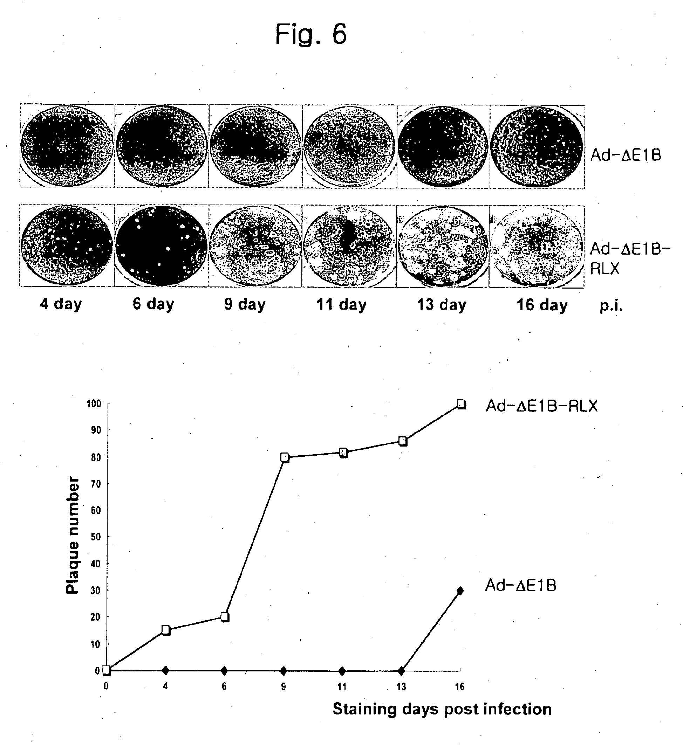

To visualize the effect of relaxin expression on the cytopathic ability and viral spread into surrounding cells, plaque formation in a solid medium containing agarose was compared. Hep3B cells were infected with Ad-ΔE1B or Ad-ΔE1B-RLX adenovirus at 3 MOI and plaque formation was then analyzed. As shown in Fig. 6, plaques were formed in shorter time for Hep3B cells infected with Ad-ΔE1B-RLX than those infected with Ad-ΔE1B. In addition, plaques formed in Hep3B cells infected with Ad-ΔE1B-RLX showed lager size than those in Hep3B cells infected with AD-ΔE1B. More specifically, with Ad-ΔE1B, plaques were observed after 16 days post-infection, whereas plaques were formed as early as 4 days post-infection for Ad-ΔE1B-RLX. These results demonstrate that relaxin-expressing adenoviruses lead to the formation of plaques in shorter period of time and much larger size owing to enhanced oncoltyic activity and viral spread to surrounding cells.

### Apoptosis induced by relaxin-expressing adenovirus

The replication incompetent adenovirus, dl-LacZ-RLX was revealed to induce the death of cells that were detached from the bottom of culture plates. Therefore, we examined whether relaxin expression is responsible for cytotoxic effect. Firstly, to determine whether relaxin induces apoptosis, flow cytometric assay was carried out after PI staining for analyzing an increase rate of subG₁ cell population containing randomly fragmented DNAs due to apoptosis.

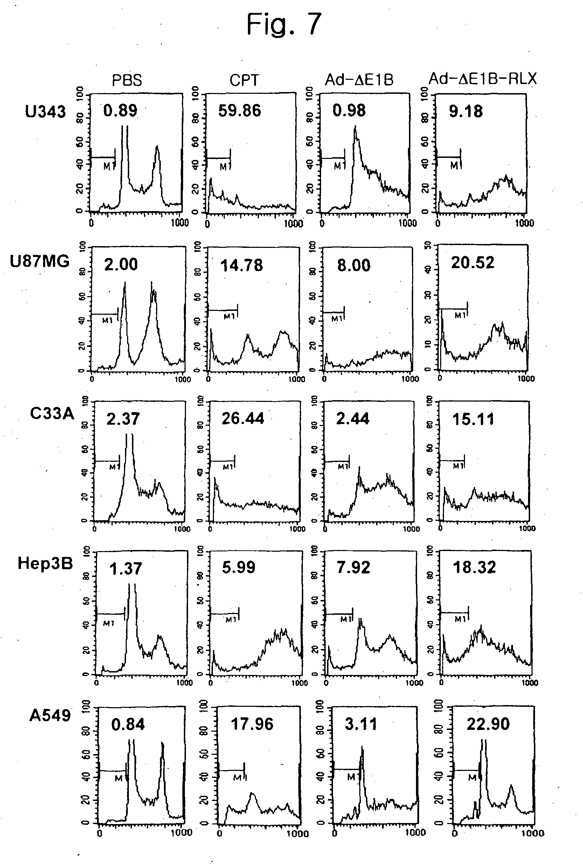

A representative of human tumor cell lines was infected with Ad-ΔE1B or Ad-ΔE1B-RLX adenovirus and harvested after 48-96 hr post-infection for measuring an increase in subG₁ cell population (Fig. 7). CPT-11 was used as a positive control for the induction of apoptosis. For A549 cells, Ad-ΔE1B induced about 3.11% of subG₁, cell copulation and Ad-ΔE1B-RLX elicited the significantly increased subG₁ cell population, 22.90%. Such increased subG₁ cell population was also observed in other cell lines (U343, U87MG, C33A and Hep3B).

Further, to accurately examine the effect of relaxin expression on cell killing potency, the progress of apoptosis induced by Ad-ΔE1B-RLX was assessed by Annexin-V and PI dual staining. Annexin-V is used to detect the translocation of phosphatidylserin (PS) to the external membrane leaflet as an early marker for apoptosis, and PI is used to identify necrosis by binding to nuclear chromatin as a late marker for apoptosis. Therefore, Annexin-V⁻/PI⁻, Annexin-V⁺/PI⁻ and PI⁺ represents healthy, apoptotic and necrotic cells, respectively.

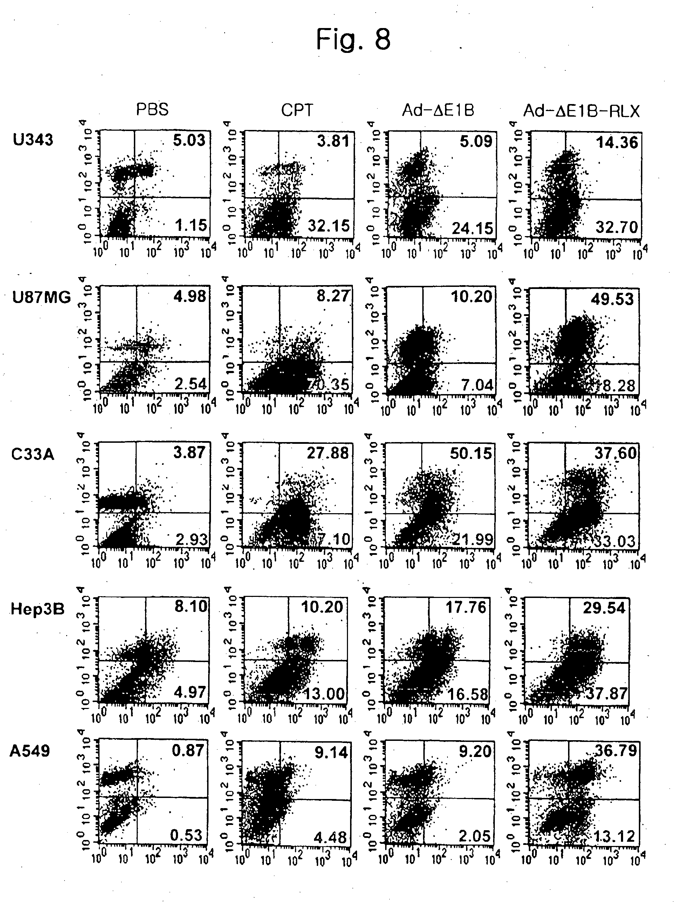

Of the CPT-treated C33A cells, 57.10% (Annexin-V⁺/PI⁻) of the cells were apoptotic, while the cells infected with Ad-ΔE1B and Ad-ΔE1B-RLX showed 21.99% and 33.03% apoptotic rate, respectively, indicating that Ad-ΔE1B-RLX adenovirus induces enhanced apoptosis rate compared to Ad-ΔE1B (Fig. 8). For other cell lines including U343, U87MG, Hep3B and A549, the relaxin-expressing adenovirus showed much higher apoptosis rate than Ad-ΔE1B adenovirus. In addition, the total of apoptosis and necrosis (Annexin-V⁺/PI⁻ and PI⁺) reflecting the entire cell death was elucidated to be much higher for Ad-ΔE1B-RLX than Ad-ΔE1B.

Collectively, these results urge us to reason that Ad-ΔE1B-RLX adenovirus elicits much higher rate of apoptosis than Ad-ΔE1B, so that the cell death occurs more frequently by Ad-ΔE1B-RLX than Ad-ΔE1B.

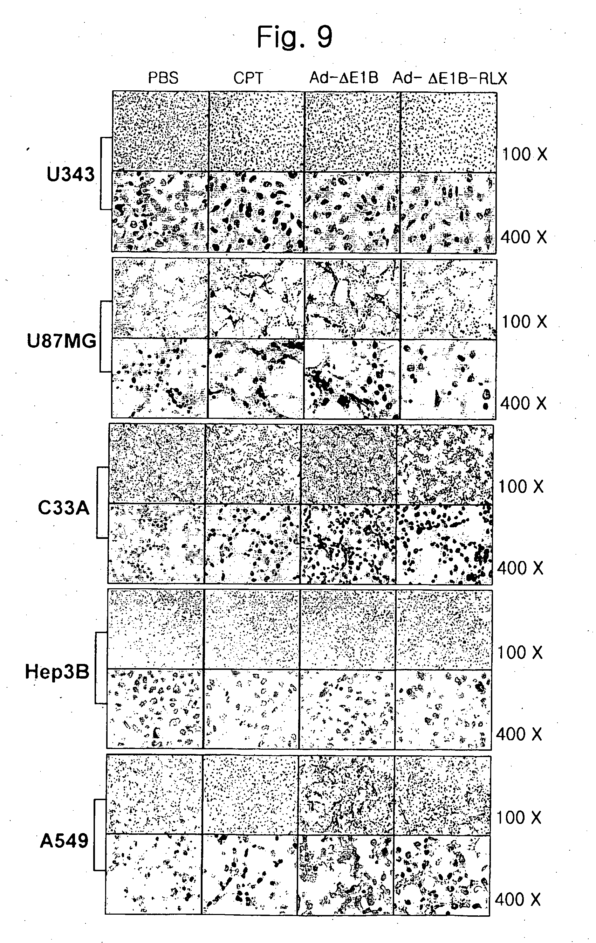

TUNNEL assay was performed for identifying DNA fragmentation as a characteristic of early apoptosis. It was shown in Fig. 9 that almost all the cells treated with CPT as a positive control were stained to dark brown, indicating the occurrence of active apoptosis. 32.5±12.5% of Ad-ΔE1B-infected U343 cells appeared light brown and 69.7±5.40% of Ad-ΔE1B-RLX-infected cells dark brown, demonstrating the higher potency of Ad-ΔE1B-RLX to induce apoptosis than Ad-ΔE1B (Table 1). Such increased apoptosis was also found in other tumor cell lines.

**TABLE 1**

| Tumor cell line | Proportion of apoptotic cells (%) | | | |
|---|---|---|---|---|
| | PBS | CPT | Ad-ΔE1B | Ad-ΔE1B-RLX |
| U343 | 10.5±5.83 | 53.5±7.45 | 32.5±12.5 | 69.7±5.40 |
| U87MG | 2.5±1.11 | 83.0±29.29 | 16.5±5.21 | 77.0±17.98 |
| C33A | 5.65±3.29 | 60.1±25.41 | 45.2±7.61 | 79.8±20.51 |
| Hep3B | 1.65±0.61 | 71.2±15.73 | 38.5±2.65 | 69.7±15.64 |
| A549 | 3.5±0.83 | 37.5±5.35 | 34.8±11.3 | 75.21±1.22 |

### Evaluation on anti-tumor effect of relaxin-expressing oncolytic a denovirus in vivo

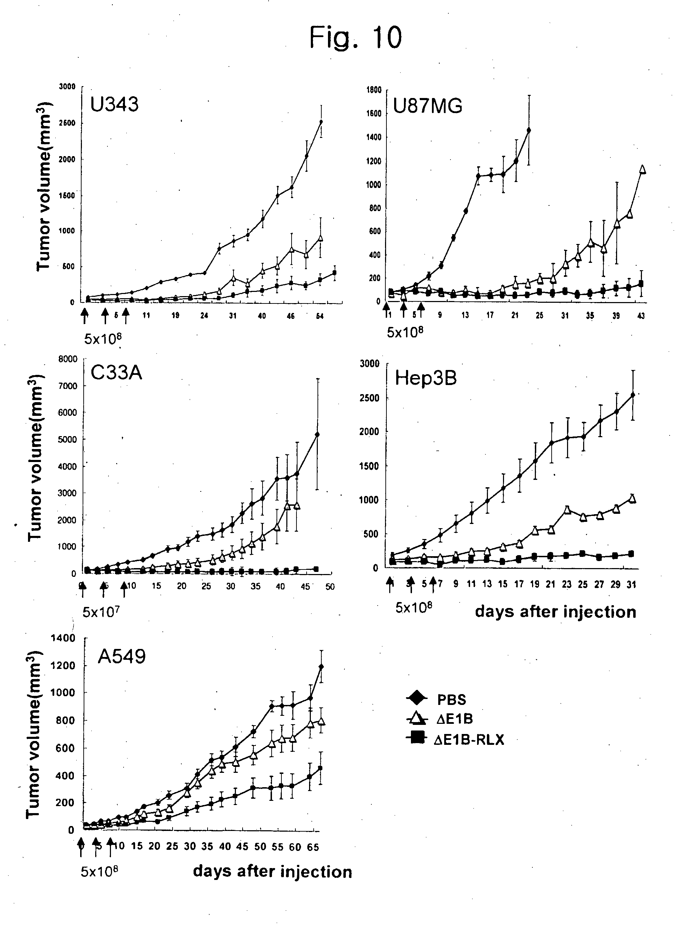

To investigate in vivo anti-tumor effect of relaxin-expressing Ad-ΔE1B-RLX, tumors xenografts formed in nude mice were infected three times every other day with Ad-ΔE1B or Ad-ΔE1B-RLX at 5 x 10⁷ - 5 x 10⁸ PFU and the growth pattern of tumors was observed. For human brain tumor U87MG, the negative control PBS resulted in the considerable growth of tumor to 1089±167.22 mm³, whereas Ad-ΔE1B and Ad-ΔE1B-RLX led to the significant suppression of tumor growth to 115.70±19.60 mm³ and 55.63±28.42 mm³, respectively (Fig. 10).

In other words, tumors treated with Ad-ΔE1B or Ad-ΔE1B-RLX adenovirus showed 10-30 fold higher anti-tumor effect than those treated with PBS. After 25 days post-treatment, all of 9 mice treated with PBS were dead. After 33 days post-infection, Ad-ΔE1B and Ad-ΔE1B-RLX adenoviruses led to the tumor volume of 399.68±96.95 mm³ and 69.51±36.73 mm³, respectively, reasoning that the relaxin-expressing adenovirus has stronger anti-tumor potency than Ad-ΔE1B. Surprisingly, Ad-ΔE1B-RLX adenovirus completely eradicated tumor in 2 mice of 7 mice at day 19 post-viral infection and wiped out tumor in 5 mice at day 41 post-infection. Also, the regrowth of tumor was not observed even after 60 days post-infection.

To examine whether such excellent anti-tumor effect of Ad-ΔE1B-RLX is also true in other human tumor cell lines, the analysis of anti-tumor effects was carried out for C33A, A549, Hep3B and U343 xenografts. The groups administered with tumor-specific oncolytic Ad-ΔE1B or Ad-ΔE1B-RLX adenovirus showed more remarkable anti-tumor effect than those treated with PBS, as shown in Fig. 10. The volume of tumor mass was much smaller for tumors treated with the relaxin-expressing adenovirus than those treated with Ad-ΔE1B. These results indicate that relaxin expression dramatically increases anti-tumor effects. In particular, C33A bearing mice treated with PBS control exhibited an average tumor volume of 2252±392 mm³ at day 32 post-treatment, as compared to Ad-ΔE1B and Ad-ΔE1B-RLX which reached an average tumor volume of 917±354 and 77±27 mm³, respectively in the same time period. In terms of regressions, at day 45 post-treatment, 25% of the mice exhibited complete regressions for Ad-ΔE1B-treated tumors as compared to 50% complete regressions seen in Ad-ΔE1B-RLX-treated mice.

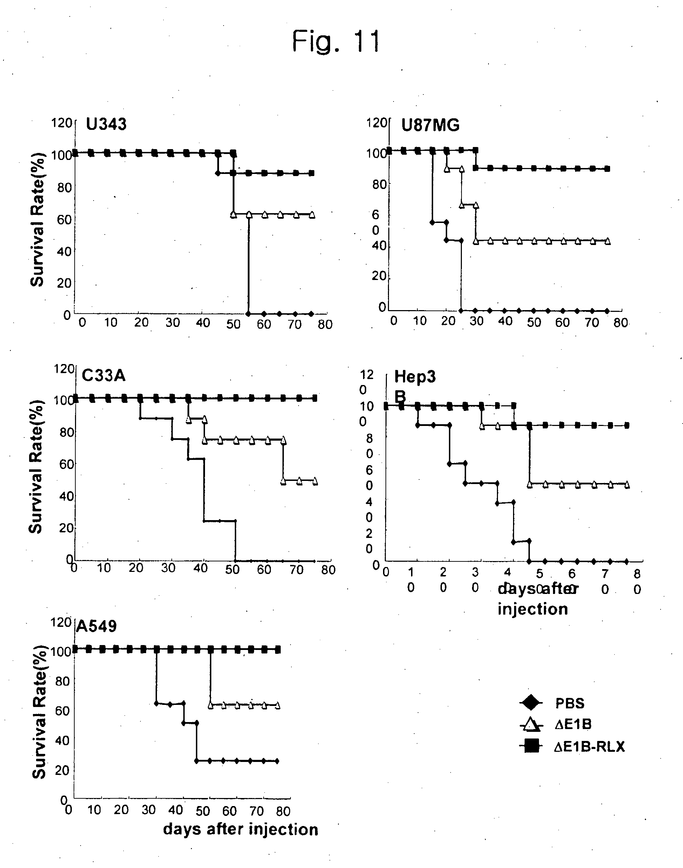

The survival rate of tumor-bearing mice was examined for the relaxin-expressing adenovirus treatment (Fig. 11). For C33A tumor bearing mice, 80 days after the beginning of the treatment, 100% of the animals treated with Ad-ΔE1B-RLX were still viable, whereas only 50% % of Ad-ΔE1B-treated mice were viable in the same time period. Furthermore, even after six months post-treatment, 50% of Ad-ΔE1B-RLX-treated animals were still completely tumor-free. Similarly, Ad-ΔE1B-RLX-induced survival benefits were obtained in all other xenograft models (U343, U87MG, Hep3B and A549). examined. In relative to each other, tumor bearing mice treated with Ad-ΔE1B-RLX survived much longer than those treated with Ad-ΔE1B in all xenograft models examined. Throughout the course of the study, no systemic toxicity such as diarrhea, loss of weight, or cachexia was observed. These results demonstrate that Ad-ΔE1B-RLX can confer significant survival benefits and tumor reduction *in vivo.*

### Change of tumor characteristics induced by relaxin-expressing replication-competent adenovirus

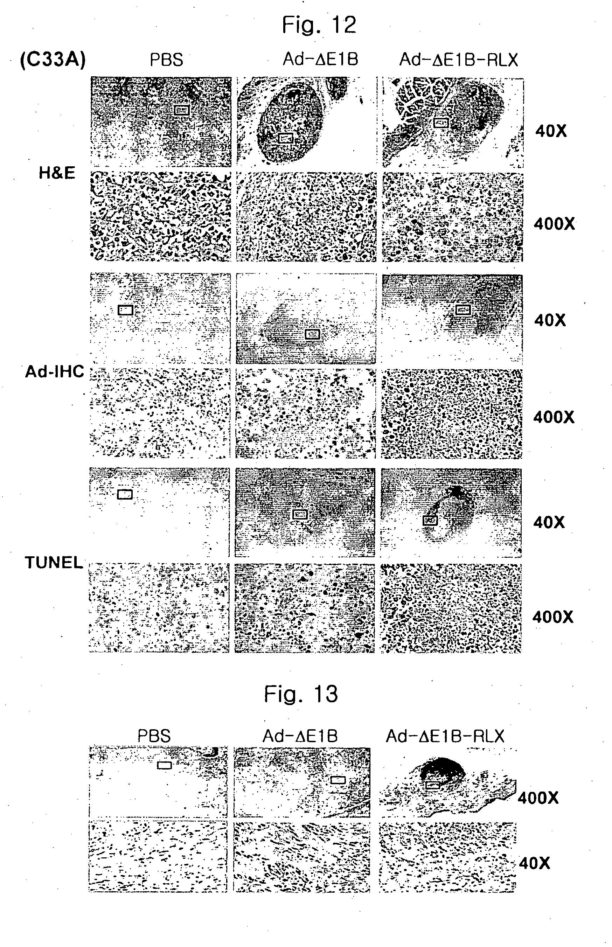

Human cervical tumor cell line C33A formed in the abdomen of nude mice was infected three times with Ad-ΔE1B or Ad-ΔE1B-RLX. Following 3 days of injection, the tumor tissues were extracted and stained with hematoxylin and eosin for histological characterization (Fig. 12). Necrotic lesions in Ad-ΔE1B-RLX-treated tumors were mainly found on the periphery of tumor mass, whereas those in Ad-ΔE1B-treated tumors were barely detectable, if any, found at the center of tumor mass.

Viral persistence and distribution within the tumor mass was then verified by immunohistochemistry using antibodies specific to adenoviral hexon protein. As shown in Fig. 12, Ad-ΔE1B-RLX adenovirus was detected mainly on the periphery of tumor that undergone necrosis. TUNNEL assay revealed that apoptosis occurred actively in the same region as necrosis. In contrast, Ad-ΔE1B induced necrosis at the center of tumor, if detectable.

Summarizing, it could be recognized that Ad-ΔE1B-RLX adenovirus replicates actively in the viral injection site, contributing to the induction of apoptosis and necrosis.

### Investigation of collagen distribution in tumor mass using Masson's trichrome staining

Human brain tumor cell line U343 formed in nude mice was injected three times with Ad-ΔE1B or Ad-ΔE1B-RLX. Following 3 days of injection, the tumor tissues were extracted and stained with Masson's trichrome to analyze the distribution of collagen (stained blue color), a major component of extracelluar matrix. trichrome stain). Tumors treated with PBS or Ad-ΔE1B consisted of high content of collagen (blue staining). In marked contrast, tumors treated with Ad-ΔE1B-RLX appeared to be devoid of collagen, indicating that relaxin expression dramatically reduced the collagen content within the tumor mass. Interestingly, tumors treated with Ad-ΔE1B-RLX were encapsulated by connective tissue, and blue-staining was only found in the boundary between tumor and normal tissue (Fig. 13).

### Inhibition of tumor metastasis by relaxin-oxpressing oncolytic adenovirus

Relaxin has been known to induce MMP expression, which can possibly increase the metastatic potential. To address this hypothesis, we evaluated the effect of relaxin-expressing oncolytic adenovirus on tumor metastasis using a spontaneous tumor metastasis model, in which B16BL6 melanoma cells were implanted subcutaneously to form a local primary tumor on the foot pad of C57BL/6 mice. Once the tumor volume reached to a volume of 100-200 mm³, animals were treated three times every other day with PBS, Ad-ΔE1B or Ad-ΔE1B-RLX. On day 5, the primary tumors were surgically removed by amputating below knee under mild anesthesia. On day 20 following primary tumor removal, the weight of metastatic tumor lesions in the lungs of the mice was measured to assess metastases to distant organs. As shown in Fig. 14, the average relative tumor burden in the lung from mice treated with Ad-ΔE1B and Ad-ΔE1B-RLX was 48±0.05 mg and 10+0.01 mg, respectively, compared to PBS-treated control group (268±0.27 mg), showing 82% and 96% inhibition, respectively. Moreover, in 4 out of 6 treated mice, both Ad-ΔE1B and Ad-ΔE1B-RLX completely inhibited the formation of metastatic lesions. These data indicate that the intratumoral injection of oncolytic adenovirus expressing relaxin in primary tumor site can greatly prevent and not enhance the formation of metastatic lesions at distal sites.

### Penetration and transduction of relaxin-expressing adenovirus into keloid

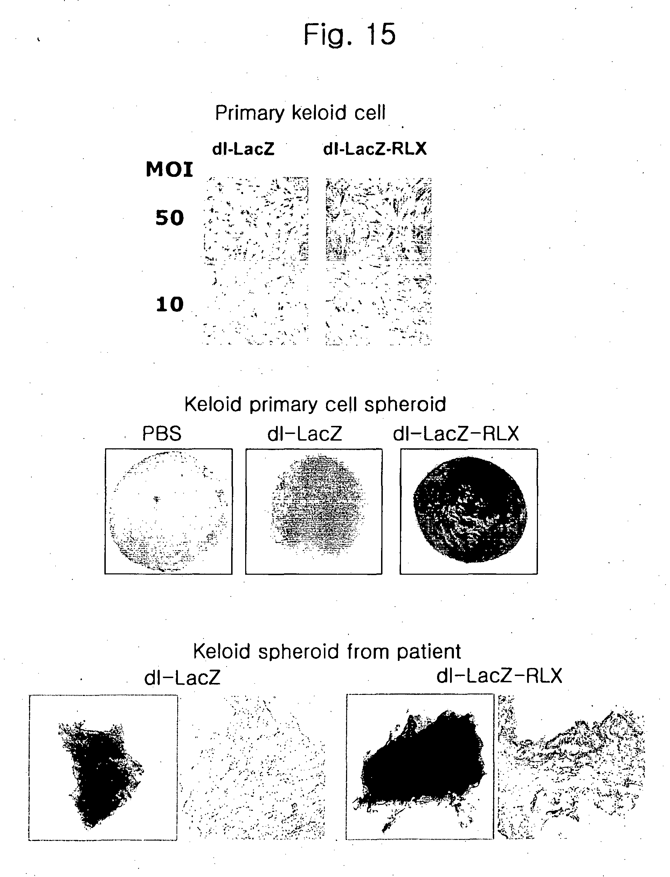

The data described previously (particularly, data for collagen distribution in tumor mass from Masson's trichrome staining) clearly demonstrate that relaxin expression dramatically reduced the collagen content within the tumor mass. Keloid is one of disorders caused by the extensive formation of extracellular matrix. To assess the therapeutic efficacy of relaxin-expressing adenoviruses on keloid, the primary keloid cell line obtained from keliod patients was infected with dl-LacZ or dl-LacZ-RLX adenovirus at an MOI of 10 or 50 and subject to X-gal staining after 2 days. As a result, the infection with dl-LacZ-RLX exhibited stronger LacZ expression than that with dl-LacZ, demonstrating that relaxin expression is responsible for the significant increase in the transduction efficiency into keloid cells (Fig. 15).

Furthermore, we examined the tissue penetration of the relaxin-expressing adenovirus using keloid cell spheroids. To verify the improved transduction efficiency of the relaxin-expressing adenovirus into keloid tissues, keloid cell spheroids prepared using primary keloid cells from keloid patients were infected with dl-LacZ or dl-LacZ-RLX adenovirus at 1 x 10⁷ PFU and subject to X-gal staining for microscopic observation. The surface of keloid cell spheroids was more intensively stained for dl-LacZ-RLX than dl-LacZ (Fig. 15).

To examine whether the enhanced transduction efficiency of dl-LacZ-RLX revealed using keloid cell spheroids is also reproducible in keloid tissues from patients, keloid tissues from patients were infected with dl-LacZ or dl-LacZ-RLX at 1 x 10⁸ PFU and subject to X-gal staining for microscopic observation (Fig. 15). While dl-LacZ-treated keloid tissues showed weak LacZ expression, dl-LacZ-RLX-treated keloid tissues were intensively X-gal stained. Furthermore, in order to assess the penetration efficiency of adenoviruses into keloid tissues, the infected tissues were were embedded in O.C.T. compound and snap frozen. The inner part of keloid tissues infected with dl-LacZ was far poorly stained as their surface. In marked contrast, for tissues infected with dl-LacZ-RLX, the distribution of LacZ expression was much more extensive and was observed throughout the entire spheroid not limited to injection site (Fig. 15).

Theses data clearly show that the transduction efficiency of the relaxin-expressing adenovirus to induce the disruption of extracelluar matrix is significantly enhanced in keloid tissues, demonstrating that the relaxin-expressing adenovirus is a promising therapeutic agent to treat keloid disorder.

### INDUSTRIAL APPLICABILITY

The present application provides a novel gene delivery system and recombinant adenovirus comprising the relaxin-encoding sequence, a gene delivering method using the gene delivery system, a pharmaceutical anti-tumor composition comprising the recombinant adenovirus, a pharmaceutical composition characterized by improved tissue penetration potency and a pharmaceutical composition for treating a disease or disorder associated with accumulation of excess extracellular matrix. According to the present invention, relaxin is responsible for the improvement in transduction efficacy and apoptotic ability to increase tumor cell killing potential dramatically.

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. Use of a relaxin for enhancing the transduction efficiency of a viral vector system, wherein the viral vector system comprises a nucleotide sequence of interest to be delivered into a cell and a nucleotide sequence encoding the relaxin, and the relaxin expressed in a cell infected with the viral vector system enhances the transduction efficiency of the viral vector system into a cell.

2. The use of a relaxin according to claim 1, wherein the cell is a cell in a tissue composed of cells interconnected each other by an extracelluar matrix.

3. The use of a relaxin according to claim 2, wherein the tissue is a tumor tissue.

4. The use of a relaxin according to claim 1, wherein the viral vector system is a recombinant adenovirus, adeno-associated virus (AAV), retrovirus, lentivirus, herpes simplex virus or vaccinia virus.

5. The use of a relaxin according to claim 4, wherein the viral vector system is a recombinant adenovirus.

6. The use of a relaxin according to claim 5, wherein the recombinant adenovirus comprises a deleted E3 or E1 region and the relaxin-encoding nucleotide sequence is inserted into the deleted E3 or E1 region.

## Patentansprüche

1. Verwendung eines Relaxins zur Verbesserung der Übertragungseffizienz eines viralen Vektorsystems, wobei das virale Vektorsystem eine Nukleotidsequenz von Interesse, die in eine Zelle eingebracht werden soll, und eine Nukleotidsequenz, die Relaxin kodiert, umfasst und das Relaxin, das in einer Zelle, die mit dem viralen Vektorsystem infiziert ist, exprimiert wird, die Effizienz der Transduktion des viralen Vektorsystems in eine Zelle erhöht.

2. Die Verwendung eines Relaxins nach Anspruch 1, wobei die Zelle eine Zelle in einem Gewebe ist, das aus Zellen gebildet wird, die miteinander durch eine extrazelluläre Matrix verbunden sind.

3. Die Verwendung eines Relaxins nach Anspruch 2, wobei das Gewebe ein Tumorgewebe ist.

4. Die Verwendung eines Relaxins nach Anspruch 1, wobei das virale Vektorsystem ein rekombinantes Adenovirus, ein Adeno-assoziiertes Virus (AAV), ein Retrovirus, ein Lentivirus, ein Herpes-simplex-Virus oder ein Vaccinia-Virus ist.

5. Die Verwendung eines Relaxins nach Anspruch 4, wobei das virale Vektorsystem ein rekombinantes Adenovirus ist.

6. Die Verwendung eines Relaxins nach Anspruch 5, wobei das rekombinante Adenovirus eine deletierte E1 oder E3 Region umfasst und die Relaxin-kodierende Nukleotidsequenz anstelle der deletierten E1 oder E3 Region eingefügt ist.

## Revendications

1. Utilisation d'une relaxine pour l'amplification de l'efficacité de la transduction d'un système de vecteur viral, où le système de vecteur viral comprend une séquence nucléotidique d'intérêt à délivrer dans une cellule et une séquence nucléotidique codant pour la relaxine, et la relaxine exprimée dans une cellule infectée avec le système de vecteur viral amplifie l'efficacité de la transduction du système de vecteur viral dans une cellule.

2. Utilisation d'une relaxine selon la revendication 1, où la cellule est une cellule dans un tissu composé de cellules interconnectées les unes avec les autres par une matrice extracellulaire.

3. Utilisation d'une relaxine selon la revendication 2, où le tissu est un tissu tumoral.

4. Utilisation d'une relaxine selon la revendication 1, où le système de vecteur viral est un adénovirus recombinant, un virus adéno-associé (AAV), un rétrovirus, un lentivirus, un virus herpès simplex ou un virus de la vaccine.

5. Utilisation d'une relaxine selon la revendication 4, où le système de vecteur viral est un adénovirus recombinant.

6. Utilisation d'une relaxine selon la revendication 5, où l'adénovirus recombinant comprend une région E3 ou E1 délétée et la séquence nucléotidique codant pour la relaxine est insérée dans la région E3 ou E1 délétée.
